(19) Europäisches Patentamt
European Patent Office
Office européen des brevets

(11) EP 2 751 129 B1

(12) EUROPEAN PATENT SPECIFICATION

(45) Date of publication and mention of the grant of the patent:
**24.01.2018 Bulletin 2018/04**

(21) Application number: **12851727.3**

(22) Date of filing: **29.08.2012**

(51) Int Cl.:
***C07K 14/335*** (2006.01) ***A61K 38/16*** (2006.01)
***A61K 35/74*** (2015.01) ***A61P 37/08*** (2006.01)
***A61P 35/00*** (2006.01)

(86) International application number:
**PCT/KR2012/006906**

(87) International publication number:
**WO 2013/077534 (30.05.2013 Gazette 2013/22)**

# (54) PROTEIN P14 WITH ANTI-CANCER AND ANTI-ALLERGY ACTIVITY AND PHARMACEUTICAL COMPOSITION COMPRISING THE SAME

PROTEIN P14 MIT KREBSHEMMENDER SOWIE ANTIALLERGISCHER WIRKUNG UND PHARMAZEUTISCHE ZUSAMMENSETZUNG DAMIT

PROTÉINE P14 AYANT UNE ACTIVITÉ ANTI-CANCÉREUSE ET ANTIALLERGIQUE ET COMPOSITION PHARMACEUTIQUE COMPRENANT CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2011 KR 20110123030**
**23.11.2011 KR 20110123033**

(43) Date of publication of application:
**09.07.2014 Bulletin 2014/28**

(73) Proprietor: **Cell Biotech Co., Ltd.**
**Gimpo-si, Gyeonggi-do 415-871 (KR)**

(72) Inventors:
- **SEO, Jae Gu**
  **Gimpo-si**
  **Gyeonggi-do 415-716 (KR)**
- **CHUNG, Myung Jun**
  **Seoul 137-070 (KR)**
- **LEE, Hyun Gi**
  **Gimpo-si**
  **Gyeonggi-do 415-060 (KR)**

(74) Representative: **Boult Wade Tennant**
**Verulam Gardens**
**70 Gray's Inn Road**
**London WC1X 8BT (GB)**

(56) References cited:
**WO-A1-2010/008113 KR-B1- 101 016 708**

- **DATABASE UniProt [Online] 16 June 2009 (2009-06-16), "SubName: Full=Uncharacterized protein{ECO:0000313|EMBL:EEI67472.1}; QVF", XP002735999, retrieved from EBI accession no. UNIPROT:C2FF76 Database accession no. C2FF76**
- **DATABASE UniProt [Online] 16 June 2009 (2009-06-16), "SubName: Full=Uncharacterized protein {ECO:0000313|EMBL:EEN80201.1}; QVF", XP002736000, retrieved from EBI accession no. UNIPROT:C2JXM4 Database accession no. C2JXM4**
- **K. MAKAROVA ET AL: "Comparative genomics of the lactic acid bacteria", PROCEEDINGS OF THE NATIONAL ACADEMY OF SCIENCES, vol. 103, no. 42, 17 October 2006 (2006-10-17), pages 15611-15616, XP055004004, ISSN: 0027-8424, DOI: 10.1073/pnas.0607117103**
- **DATABASE GENBANK [Online] 19 October 2011 XP003032558 Database accession no. YP_806775.1**
- **DATABASE GENBANK [Online] 10 November 2010 XP003032559 Database accession no. ZP_03210516.1**
- **LEE, J. W. ET AL.: 'Immunomodulatory and antitumor effects in vivo by the cytoplasmic fraction of Lactobacillus casei and Bifidobacterium longum.' J. VET. SCI. vol. 5, no. 1, 2004, pages 41 - 48, XP001526218**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to protein p14 which exhibits anti-cancer and anti-allergy activity, and a pharmaceutical composition for the treatment, management, alleviation, improvement or prevention of cancer and/or allergic diseases, comprising the same.

### Background Art

Cancer is a disease which is the main cause of human deaths around the world. Although the survival of cancer patients has increased with the development of new types of chemotherapy, neither the onset nor the severity of cancer has decreased.

Cancer is an aggregate of cells characterized by unregulated cell growth and proliferation due to the loss of differentiation ability. Carcinogenesis is explained by a multistep process involving 5~8-steps of alterations of oncogenes and tumor suppressor genes. The activation of an oncogene induces the aberrant proliferation of cells whereas a tumor suppressor gene, when activated, initiates the operation of a cell death program in which specific cells are programmed to perish, thereby preventing cells from becoming cancerous.

Because they are adapted to kill the cells that rapidly proliferate, most of the currently used anti-cancer agents cannot selectively remove cancer cells, but have an inhibitory influence on the normal cells that outgrow cancer cells, like the epithelial cells of digestive organs, or dermal papilla cells. Chemotherapy with such anti-cancer agents damages the normal cells as well, with the concomitant generation of side effects such as vomiting, hair loss, etc. In addition, conventional anti-cancer agents destroy lymphocytes, which rapidly proliferate during an immune response to pathogens. Accordingly, persons who are receiving chemotherapy are susceptible to bacterial or viral infections and have rather a decreased potential to control cancer cells. Further, the functioning of their bone marrow is weakened because conventional anti-cancer agents induce myelosuppression.

There is therefore a pressing need for an anti-cancer agent that is effective at preventing the onset of cancer as well as treating cancer, with minimal toxicity.

About 20% of the world population suffers from allergic disorders including asthma, allergic rhinitis, food allergy, atopic dermatitis and hypersensitivity. Anti-histamine agents or steroid drugs, used as therapeutics for allergy, may incur significant side effects. Thus, research has been made to develop a therapeutic for allergy disorders which is therapeutically effective without side effects.

### Disclosure of Invention

### Technical Problem

It is therefore an object of the present invention to overcome the problems encountered in the prior art and to provide a novel protein having anticancer activity.

It is another object of the present invention to provide a pharmaceutical composition for the prevention or treatment of cancer, which exhibits high therapeutic efficiency with side effects minimized.

It is a further object of the present invention to provide a food composition useful as an aid in the treatment or prevention of cancer.

It is still a further object of the present invention to provide a novel protein having an anti-allergy activity.

It is still another object of the present invention to provide a food composition useful as an aid for the treatment, management, alleviation, improvement or prevention of allergic diseases.

### Solution to Problem

The present invention provides a pharmaceutical composition for use in the treatment of allergy, comprising:

(a) the protein consisting of the amino acid sequence of SEQ ID NO: 1 or 3, and
(b) a pharmaceutically acceptable excipient, diluent, carrier or buffer.

In certain embodiments the protein is derived from *Lactobacillus casei, Lactobacillus paracasei or Lactobacillus rhamnosus.* In certain embodiments the protein has a molecular weight of 14 kDa.

## Advantageous Effects of Invention

As described hitherto, the present description addresses a pharmaceutical composition for the treatment or prevention of cancer, comprising a novel protein derived from lactic acid bacteria, as an active ingredient. In addition to exhibiting excellent preventive or therapeutic effects on cancer, the composition creates neither of cytotoxicity nor causes side effects because a protein derived from probiotic organisms or dead bacteria beneficial to humans is used as an active ingredient. Further, the active ingredient can maintain its pharmaceutical effects for a long period of time. Thus, the composition may be safely applied to a food composition in addition to a pharmaceutical composition.

The present invention addresses a composition for the prevention or treatment of allergic diseases, comprising a novel protein derived from Lactobacillus as an active ingredient. Protein p14, used in the present invention, induces the synthesis of IFN-γ and regulates the production of IL-4, thus exhibiting preventive or therapeutic effects on allergic disease. Derived from probiotic organisms or dead bacteria beneficial to humans, the active ingredient is free of cytotoxicity and does not cause side effects. In addition, its pharmaceutical efficiency lasts for a long period of time. Thus, it is safely applicable to a food composition in addition to a pharmaceutical composition.

Moreover, the present invention provides a gene coding for the protein p14 which has anti-allergy and/or anti-cancer activity, with which recombinant protein p14 can be produced in a large quantity.

## Brief Description of Drawings

The above and other objects, features and other advantages of the present invention will be more clearly understood from the following detailed description taken in conjunction with the accompanying drawings, in which:

FIG. 1 is an FPLC chromatogram showing 10 ~ 100 kDa fractions of a culture of *Lactobacillus paracasei;*
FIG. 2 is a graph showing anti-cancer activity of fraction 16-19 of a culture of *Lactobacillus paracasei;*
FIG. 3 shows the identification of proteins having anticancer activity as assayed by silver staining;
FIG. 4 shows Blast search results for the identification of anticancer proteins through amino acid sequence alignment;
FIG. 5a shows schematic structural diagrams of primers designed on the basis of deduced CDS areas;
FIG. 5b shows PCR products of genes coding for 13 kDa, 14 kDa, and 49 kDa proteins which will be inserted into E. coli expression vectors;
FIG. 6 shows the cloning of genes coding for 13 kDa, 14 kDa, and 49 kDa proteins into vectors, using restriction enzymes and T4 ligase;
FIG. 7 shows SDS-PAGE results of recombinant proteins overexpressed in E. coli colonies by IPTG induction;
FIG. 8 shows the expression of 13 kDa, 14 kDa, and 49 kDa proteins over time in E. coli colonies under IPTG induction;
FIGS. 9a and 9b show isolation, purification and dialysis results of protein p14 from E. coli cultured on a large scale;
FIG. 10 shows similarity between the recombinant proteins P13 and p14 in terms of amino acid sequence;
FIGS. 11a and 11b show anti-cancer activity of P13 and p14 against colorectal cancer cell lines HT-29 and DLD-1, as measured by MTT assay;
FIG. 12 shows the cytotoxicity of protein p14 in terms of the cell survivability of mouse macrophages;
FIGS. 13a and 13b shows immunopotentiation of protein p14 in mouse splenocytes;
FIG. 14 shows feed intake of F344/N mice treated with or without p14 after the induction of large intestine cancer by the carcinogen DMH;
FIGS. 15a and 15b show counts of AC (aberrant crypt) and ACF (aberrant crypt foci) formed in the colonic mucosa of mice treated with or without protein p14 after cancer induction by DMH;
FIG. 16 shows the growth of tumors in nude mice xenografted with the human large intestine cancer cell line DLD-1, which is inhibited 10% more by p14 than by the control;
FIG. 17 shows sizes of tumors in nude mice xenografted with the human large cancer intestine DLD-1 before administration with p14 and after four rounds of administration with p14;
FIG. 18a shows INF-γ levels in activated mouse splenocytes treated with the protein p14 of the present invention, and FIG. 18b shows IL-4 levels in activated mouse splenocytes treated with the protein p14 of the present invention;
FIGS. 19a to 19c show anti-allergy activity of the protein p14 of the present invention in terms of NO production in mouse macrophages over time;
FIG. 20 shows in vivo anti-allergy effects of p14 on atopy-induced mice; FIG. 21 shows serum IgE levels in atopy-induced mice after treatment with the protein p14 of the present invention; and
FIG. 22 shows serum IL-4 levels in atopy-induced mice after treatment with the protein p14 of the present invention.

## Best Mode for Carrying out the Invention

As used herein, the term "cancer" is intended to include tumors, neoplasms, and malignant tissues or cells.

Examples of the cancer include cancer of the large intestine, lung cancer, small cell lung cancer, stomach cancer, liver cancer, blood cancer, bone cancer, pancreatic cancer, skin cancer, head and neck cancer, intradermal or intraocular melanoma, uterine cancer, ovarian cancer, rectal cancer, perianal cancer, colon cancer, breast cancer, fallopian tube carcinoma, endometrial cancer, uterine cervical cancer, vaginal cancer, vulvar cancer, Hodgkin's disease, esophageal cancer, small intestine cancer, endocrine gland cancer, thyroid cancer, parathyroid cancer, adrenal cancer, soft tissue sarcoma, urethral cancer, penis cancer, prostate cancer, chronic or acute leukemia, lymphoma, bladder cancer, renal caner, ureteral cancer, renal cell carcinoma, renal pelvis cancer, CNS tumor, primary CNS lymphoma, spinal cord tumor, brainstem glioma, pituitary adenoma and a combination thereof.

The term "allergy," as used herein, means INF- $\gamma$ mediated allergy. In this regard, rhinitis, sinusitis, atopy, asthma, hypersensitive pneumonia, extrinsic allergic alveolitis, conjunctivitis, hives, pruritus, sun allergy, eczema, dermatitis, anaphylaxis, angioedema, allergic headache and migraine, and certain digestive troubles fall within the scope of the term allergy.

Provided herein is an anti-cancer protein p14 having the amino acid sequence of SEQ ID NO: 1 or 3. Also provided is a pharmaceutical composition for the treatment of cancer, comprising the protein p14 as an active ingredient.

Protein p14 has a molecular weight of about 14 kDa. The composition of the present invention may comprise a functional equivalent of protein p14. As used herein, the term "functional equivalent" is intended to refer to a protein which shares an amino acid sequence homology of 70% or greater, preferably 80% or greater and more preferably 90% or greater with the SEQ ID NO: 1 or 3 as a result of amino acid residue addition, substitution or deletion, exhibiting a physiological function substantially identical to that of the protein of SEQ ID NO: 1 or 3. The term "substantially identical physiological function," as used herein, means anti-cancer and/or anti-allergy activity in animals.

In one embodiment of the present invention, protein p14 is derived from *Lactobacillus casei, Lactobacillus paracasei or Lactobacillus rhamnous.* Illustrative, non-limiting examples include Lactobacillus paracasei LPC4 (KCTC 11866BP). The protein p14 derived from Lactobacillus rhamnosus has the amino acid sequence of SEQ ID NO: 3, which shares a homology of about 90% with that of SEQ ID NO: 1 and which can be encoded by the nucleotide sequence of SEQ ID NO: 4.

The protein p14 derived from *Lactobacillus casei, Lactobacillus paracasei or Lactobacillus rhamnosus* is recognized by the epidermal growth factor receptor (EGFR), present on the cell surface, to enhance the Akt-mediated signaling pathway or the JNK- or MAPK-mediated signaling pathway.

Functioning to increase the release of interferon gamma and suppress the production of interleukin-4, protein p14 can be used for preventing or treating allergic diseases.

Also provided herein is an anticancer pharmaceutical composition comprising a gene (nucleic acid) coding for the protein p14 as an active ingredient. The gene includes genomic DNA and cDNA so long as it encodes protein p14. Preferably, the gene comprises the nucleotide sequence of SEQ ID NO: 2 or 4.

Also, a mutant of the nucleotide sequence may fall within the scope of the present invention. For example, the gene may have a nucleotide sequence which shares a homology of 70% or higher, preferably 80% or higher and more preferably 90% or higher with the nucleotide sequence of SEQ ID NO: 2. The term "percentage of sequence homology" as used herein in connection with a polynucleotide refers to a value determined by comparing two optimally aligned sequences over a comparison window, wherein the portion of the polynucleotide sequence in the comparison window, wherein the portion of the polynucleotide sequence in the comparison window may comprise additions or deletions (i.e., gaps) as compared to the reference sequence (which does not comprise additions or deletions) in order to optimally align of the two sequences.

Also provided herein is a recombinant expression vector carrying a protein p14-encoding gene (nucleic acid). The recombinant expression vector may be constructed by linking a gene of interest to a recombinant vector used in the art in a conventional manner. This expression vector may be a plasmid or a viral vector. Plasmid expression vectors are approved for use in gene delivery to humans by the FDA. Plasmid expression vectors can deliver DNA directly to human cells (Nabel, E. G., et al., Science, 249: 1285-1288, 1990). Unlike viral vectors, plasmid DNA can be advantageously purified with homogeneity. Mammalian expression plasmids known in the art may be used as expression vectors in the present invention. Examples of the expression vector useful include, but are not limited to, pRK5 (EP No. 307,247), pSV16B (PCT Patent Publication No. WO 1991/08291) and pVL1392 (PharMingen).

The viral expression vector carrying the nucleic acid, useful in the present invention, may include those derived from a retrovirus, adenovirus, herpes virus and avipox virus, but is not limited thereto.

Also provided herein is a transformant prepared by introducing the recombinant vector into a host cell. The transformation may be achieved using a conventional method. For example, a recombinant vector can be introduced into target cells using transient transfection, microinjection, transduction cell fusion, calcium phosphate precipitation, liposome-mediated transfection, DEAE dextran-mediated transfection, polybrene-mediated transfection, electroporation, a gene gun or a well-known method (Wu et al., J. Bio. Chem., 267:963-967, 1992; Wu and Wu, J. Bio. Chem., 263:14621-14624, 1988).

No particular limitations are imposed on the host cells to be formed into transformants so long as they can be suitably transformed by the recombinant vectors of the present invention. Various cells known in the art, whether natural or synthetic, may be employed. For example, E. coli BL21 and BL21 (DE3) may be used, with a preference for E. coli BL21 (DE3). The transformed E. coli can be cultured using a method that is commonly used to culture E. coli in the art.

Also provided herein is an anti-cancer pharmaceutical composition, comprising protein p14 having the amino acid sequence of SEQ ID NO: 1 or 3 as an active ingredient. The anti-cancer pharmaceutical composition may comprise a pharmaceutically acceptable excipient, diluent, carrier or buffer in addition to the active ingredient protein p14, which exhibits anti-cancer activity.

The present invention addresses an anti-allergy pharmaceutical composition comprising (a) protein p14, having an anti-allergy activity; and, (b) a pharmaceutically acceptable excipient, diluent, carrier or buffer.

For pharmaceutically acceptable additives suitable for use in the present invention, including buffer, diluents, vehicles, supplements, and other excipients, Remington's Pharmaceutical Sciences (19th ed., 1995) may be referred to. Suitable agents may be employed in the composition to achieve various other purposes. For example, a buffer, preservatives, co-solvents, oils, wetting agents, softeners, stabilizers or antioxidants may be used. Examples of aqueous preservatives useful in the present invention include sodium bisulfate, sodium thiosulfate, benzalconium chloride, chlorobutanol, thimerosal, ethyl alcohol, methyl paraben, polyvinylalcohol, benzyl alcohol and phenylethyl alcohol. These agents may be present in an amount of about 0.001 to about 5 wt% individually. Aqueous buffers suitable for use in the present invention are sodium carbonate, sodium borate, sodium phosphate, sodium acetate and sodium bicarbonate, the desired routes of administration of which have been approved by the FDA. These agents may be present in an amount sufficient to keep the pH of the system within the range of about 2 to about 11. Thus, the buffer may be used in an amount of about 5 wt % based on the total weight of the composition. In addition, electrolytes such as sodium chloride and potassium chloride may be contained in formulations.

Medical products to which the protein p14 of the present invention is applied to may further comprise one or more active ingredients exhibiting anticancer activity identical or similar to that of the protein p14.

For clinical use, the protein p14 may be orally administered and may be formulated into general medical preparations. In practical clinics, the protein p14 of the present invention may be used in various oral dosage forms. Solid forms for oral administration include tablets, pills, powders, granules, capsules, etc.

The method for preparing the anticancer and/or anti-allergy pharmaceutical composition comprises formulating protein p14 with a pharmaceutically acceptable excipient, diluents, carrier or buffer. In one embodiment, the preparation method of the present invention comprises formulating protein p14, at least one anticancer agent and a pharmaceutically acceptable excipient, diluent, carrier, or buffer. Examples of the anticancer agent include ciplatin, doxorubicin and etoposide, but are not limited thereto.

According to a method well known to those skilled in the art, the pharmaceutical composition of the present invention may be formulated, together with pharmaceutically acceptable carriers and/or excipients, into unit dose forms or may be included within a multiple dose package.

The anticancer pharmaceutical composition or the anti-allergy pharmaceutical composition of the present invention comprises a pharmaceutically effective amount of protein p14. The term "pharmaceutically effective amount," as used herein, refers to an amount sufficient to treat or prevent cancer. The pharmaceutically effective amount of protein p14 in accordance with the present invention is within the range of 0.1 ~ 100 mg/day/ kg of body weight, and preferably 12 mg/day/kg of body weight. The dose of protein p14 may vary depending on various factors including the route of administration, the severity of illness, and the gender, weight and age of patient.

In accordance with another embodiment, the composition comprising protein p14 of the present invention may be applied to foods including beverages helpful in the treatment of cancer and/or allergic diseases. Foods to which the protein p14 of the present invention is applicable are beverages, teas, vitamin complex products, health aid foods, etc. The protein p14 itself, isolated from lactic acid bacteria, creates almost no toxicity and side effects, so that it can be safely used for a long period of time for prophylactic purposes.

**Mode for the Invention**

A better understanding of the present invention may be obtained through the following examples which are set forth to illustrate, but are not to be construed as the limit of the present invention.

EXAMPLES

EXAMPLE 1: Isolation and Identification of Protein p14 and Production of Recombinant Protein

1-1. Isolation and Purification of Protein with Anticancer Activity from a Culture of

*Lactobacillus paracasei*

Proteins were separated into elution fractions on a phenyl sepharose column loaded with a culture of the Lactobacillus paracasei LPC4 strain (KCTC 11866BP). For this, first, the Lactobacillus paracasei strain was cultured and concentrated. Proteins with anticancer activity were purified from the cell concentrate by FPLC (fast protein liquid chromatography, Sephadex 75) on a Sephadex 75 column (fractionation range Mr = 3,000 ~ 70,000) eluting with a buffer (0.05 M phosphate and 0.15 M NaCl, pH 7.2) at a flow rate of 0.5mL/min, with 1 mL of the sample (2~4 mg/mL) loaded thereinto. Proteins with anticancer activity were found in 3~100 kDa and 10~30 kDa fractions after ultra filtration (FIG. 1).

1-2. Determination of Anticancer Activity Fractions and Identification of Amino Acid Sequences thereof

Fraction Nos. 16~19 were assayed for anticancer activity. In this regard, 900 μL aliquots of each of the cancer cell lines including stomach cancer (AGS), lung cancer (A549), breast cancer (MCF-7), ovarian cancer (SKOV-3) and colorectal cancer (LoVo) were each put into a well of a 24-well plate and incubated for 24 hours (37°C, 5% CO2). To each well was added 100 μL of each of the purified fractions before incubation for an additional 24 hours (37°C, 5% CO2). Subsequently, each well was supplemented with 100 μL of an MTT solution (0.5mg/mL) and incubated for 4 hours (37°C, 5% CO2). After removal of the supernatant, 300 μL of DMSO (dimethyl sulfoxide) was added to each well to dissolve the produced formazan, followed by measuring absorbance at 540 nm in an ELISA reader. From the absorbance values, inhibition activity of the protein fractions against cancer cell growth were calculated according to the following equation and expressed as inhibition ratio (%). The results are shown in FIG. 2. Each fraction was recovered in an amount of 3 mL/fraction from FPLC (Sephadex 75) (0.5ml/min).

$$\text{Inhibition ratio}(\%) = (1\text{-}T/C)X100$$

(T: absorbance in the presence of sample, C: absorbance in the absence of sample)

Proteins in the 3~100 kDa fractions were examined for anticancer activity. As can be seen in FIG. 2, the control did not exhibit cytotoxicity against the cancer cells whereas proteins in fraction Nos. 16 to 19 of the 10~30 kDa fractions were found to have anticancer activity.

1-3. Identification of Anticancer Protein p14

Proteins were separated at p14 and p44 from fraction Nos. 16~19, as assayed by silver staining and Coomassie blue 250 staining (FIG. 3). They were characterized and their amino acid sequences were determined. In this context, liquid chromatography/ mass spectroscopy of the proteins detected by staining yielded three peaks at p49, p14 and p13. The 49-kDa protein is a cell wall-associated hydrolase and showed a similarity of 95 % or higher with a cell wall-associated hydrolase. The protein p14 is a hypothetical protein whose function remained unknown, with a similarity of 95% or higher with a hypothetical protein LSEI_0419(FIG. 4).

1-4. Production of Recombinant Proteins

A. Design and Construction of Restriction Site-Containing Primers

To clone genes coding for the 13 kDa, the 14 kDa and the 49 kDa protein into the E. coli expression vector pRSET A (Invitrogen), primers containing BamHI and XhoI sites were designed and synthesized as shown in FIG. 5a. In preparation for counteracting the likelihood of not recognizing the restriction sites due to mutation, one or two bases were added in close proximity to the restriction sites. The reading frame was also considered when designing the primers. The genes coding for 13 kDa, 14 kDa and 49 kDa proteins were amplified using the primers and cloned into a pRSET A vector (FIG. 5a).

B. PCR for Amplification of CDS Area to Be Inserted into E. coli Expression Vector

Using the primers, the PCR products of the CDS areas were amplified with annealing at 55°C. For amplification, a reaction mix containing 3 μL of a substrate, (10 ng/μL), primers (10 pM), MgCl2 (25mM), dNTP(2.5mM), Taq polymerase (5 U/μL) and 10x buffer was employed. PCR started with initial denaturation at 95°C for 2 min and was performed with 30 thermal cycles of denaturing at 95°C for 20 sec, annealing at 55°C for 35 sec and extension at 72°C for 20 sec. High fidelity polymerase Taq (Invitrogen) was employed for the PCR, with the DNA of *Lactobacillus casei* serving as a template. The PCR products were measured to be 369, 372, and 1494 bp long, which were the same as the sizes of the three proteins, respectively (FIG. 5b).

C. Transformation

pRSET and the PCR products were digested with the restriction enzymes BamHI, XhoI and EcoRI at 37°C for 2 hours, and separated on agarose gel by electrophoresis. The vector was ligated with the inserts. For this, the vector was used at an intensity ratio of 1: 3 with the insert as measured by EtBr staining. The ligated vector was transformed into competent cells (DH5α) and incubated to form a total of 22 colonies (FIG. 6). Of them, nine (colony Nos. 6, 7, 8, 10, 11, 13, 18, 20 and 21) were found to have exact inserts. Base sequencing analysis was performed on 13 kDa, 14 kDa, and 49 kDa recombinant protein-expressing plasmids thus obtained, using a T7 promoter primer to confirm the transformation.

D. Expression of 13 kDa, 14 kDa, and 49 kDa Recombinant Proteins

After the BL21 strain was transformed with the recombinant plasmid carrying a gene coding for the 13 kDa, the 14 kDa or the 49 kDa protein, a selection was made of E. coli colonies that showed a high expression level of the protein. To this end, 7 or 8 colonies were induced for 4 hours in the presence of 1 mM IPTG to express the proteins, followed by electrophoresis on 15% bisacrylamide gel for 90 min at 80 V. (Loading volume: 1 mL induction, 25 μL 1X sample buffer Boiling 5 min, 10 μL loading). As a result, high levels of the proteins were detected in colony Nos. 13-1, 14-4 and 49-8 (FIG. 7).

E. Induction of Selected Colonies Under Various Conditions

The 14 kDa recombinant protein was observed to be stably expressed after having been induced for 1 hour with IPTG, irrespective of the concentration of IPTG. The colonies were O/N cultured in LB broth. Of 3 mL of the O/N culture, 0.5 mL was inoculated into 10 mL of fresh LB broth. The cells were grown for two or more hours to an OD600 of 0.5 before induction with IPTG. Samples were taken by time, and then run on 15% bisacrylamide gel for 90 min at 80 V by electrophoresis (Loading volume: 1 mL induction, 25 μL 1X sample buffer, Boiling 5 min, 10 μL loading). Induction of the proteins of 13 kDa, 14 kDa, and 49 kDa over time showed that expression of only the 14 kDa recombinant protein was stable (FIG. 8).

F. Isolation and Purification of p14 Recombinant Protein from Large-Scale Culture

Based on the result that only expression of the protein p14 was stable (10 mL culture, 1 mM IPTG), the culture was scaled up (300 mL) and stable induction was made therein (0.1 mM IPTG) (FIG. 9a). In addition, cells were harvested from the 300 mL culture and proteins were recovered from the cells using the BugBuster Master Mix (Merk). Purification using the His Taq BugBuster NI-NTA His*Bind Purification Kit (Merk) gave 2 mL of a purified protein. SDS-PAGE of 20 μL of the purified protein demonstrated purification. In this regard, purification was confirmed by running a flow through the His Taq column, running a wash fraction, and separating an elution fraction on the gel and visualizing with Coomassie blue 250. The protein p14 recovered many times in the same manner was observed to have a high purity after dialysis (FIG. 9b).

G. Analysis of Amino Acid Sequence of p14 Recombinant Protein (FIG. 10)

After electrophoresis on Coomassie gel, the band of the p14 recombinant protein expressed in E. coli was recovered and stirred for 5 min in 100 μL of a destaining solution (50% MeOH/D.W.). Then, the destaining solution was completely removed. The residue was incubated for 20 min in 100 μL of 200 mM ABC (ammonium bicarbonate, pH 7.8) and then, the ABC was removed. The residue was vortexed for 2 min in 100 μL of acetonitrile and then, the acetonitrile was discarded. Again, the residue was vortexed for 2 min in 100 μL of ABC and then the ABC was discarded. Vortexing was also continued for 2 min with 100 μL of acetonitrile. This procedure was repeated three times in total. After acetonitrile was completely removed in a final step, the gel was dried at room temperature. To the dried gel was added 20 μL of trypsin (0.2 μg) which was incubated for 45 min in ice. The trypsin which remained unabsorbed into the gel was removed. The gel was incubated at 37°C for 12 hours in 70 μL of 50 mM ABC. GELoader Tip microcolumns packed with Poros R2 resins were prepared. A. 10 mL syringe was fitted to the tip so as to apply air pressure to the column. The microcolumns were equilibrated with 20 μL of 5% formic acid solution which was allowed to pass through the microcolumn by applying them using the syringe (equilibration). To the equilibrated microcolumns was loaded 30 μL of a solution of the peptide obtained by in-gel digestion (loading). Using the syringe, 20 μL of 5% formic acid solution was passed through the microcolumns (washing). Thereafter, the peptide was eluted with 1.5 μL of 50% methanol/49% H2O/1% formic acid solution by applying air pressure from the syringe (elution). With the aid of nano-electrospray needle (EconoTipTM, New Objective, USA), 1.5 μL of the eluate was applied to a Q-TOF source. The amino acid sequence of the separated proteins was examined using an MS instrument: Hybrid Quadrupole-TOF LC/MS/MS Mass Spectrometer (AB Sciex Instruments, CA 94404 USA). Three proteins 10, 14, and 14 were found to have the same amino acid sequence as that of p14 (conducted by Protein Works).

EXPERIMENTAL EXAMPLE 1: In vitro Anticancer Activity Assay (MTT assay)

Each of the colorectal cancer cell lines DLD-1 and HT-29 were plated into 96-well plates and incubated for 48 hours with various concentrations of the protein p14 (10 ng/ml to 1000 ng/ml). For a negative control, the cells were treated only with 0.1% PBS alone while protein P13 isolated from lactic acid bacteria was used as a positive control. Then, the cells were disrupted in a lysis buffer. The cell lysate was incubated at 37°C for 50 min with MTT (3-(4,5-dimethylthiazol)-2,5-diphenyltetrazolium bromide, Sigma, U. S. A.). Absorbance at 610 nm was measured in a microplate reader (Benchmark, BIO-RAD, Japan) to calculate cell survivability. The results are shown graphically in FIGS. 11a and 11b, which are for HT-29 and DLD-1, respectively. As can be seen in FIGS. 11a and 11b, protein p13 did not exhibit inhibitory activity against the cancer cells whereas protein p14 was excellent at inhibiting the growth of both DLD-1 and HT-29 cell lines in a dose-dependent manner at an inhibition rate of from about 20% to about 40%.

EXPERIMENTAL EXAMPLE 2: Cytotoxicity Assay

To examine whether the protein p14 of the present invention is toxic to cells, an MTT assay was performed. After being overexpressed, the protein p14 was assayed for cytotoxicity against the mouse marcrophage cell line Raw264.7 while using P13 as a positive control. The cells were seeded at a density of 5×103 cells/well into 96-well plates, and P13 and p14, purified after overexpression, were added to each well (at a concentration of from 10 ng/ml to 1000 ng/ml). After incubation for 48 hours, the cells were lyzed in a lysis buffer and incubated at 37°C for 50 min with MTT (3-(4,5-dimethylthiazol)-2,5-diphenyltetrazolium bromide, Sigma, U. S. A.). Absorbance at 610 nm was measured using a microplate reader (Benchmark, BIO-RAD, Japan) to calculate cell survivability. The results are shown in FIG. 12. As can be seen in FIG. 12, there is no difference in cell survivability between the test group and the control. Thus, protein p14 was found to have no cytotoxicity, as measured by MTT assay (FIG. 12).

EXPERIMENTAL EXAMPLE 3: Immunopotentiation of Protein p14

An examination was made of whether the protein p14 of the present invention has anti-cancer immunoactivity. Balb/c splenocytes were seeded at a density of 2x106 cells/mL into 96-well plates and treated for 48 hours with various concentrations of the target proteins P13 and p14 (0.01 μg/mL to 10 μg/mL). After centrifugation, the supernatant thus obtained was analyzed using ELISA. A negative control was treated with 0.1% PBS devoid of protein p14 while a positive control was treated with P13. Concentrations (pg/mL) of the cytokines INF-γ and IL-12 were measured and are shown in FIGS. 13a and 13b, respectively. As is understood from the data of FIGS. 13a and 13b, protein p14 was observed to induce the expression of IL-12 and INF-γ at higher rates than did the positive control P13, indicating that protein p14 has efficient anticancer immunoactivity.

EXPERIMENTAL EXAMPLE 4: In vivo Anticancer Activity Assay (MTT assay)

4-1. Induction of Neoplastic Polyps by 1.2-Dimethylhydrazine (DMH)

A solution of the carcinogen 1,2-dimethylhydrazine (DMH; Sigma, USA) in citrate buffer was subcutaneously injected at a dose of 30 mg/kg four times (twice a week for two weeks) into the head and neck area of F344/N rats to form neoplastic polyps which developed into colorectal cancer. The test material protein p14 was mixed with powdered basic feedstuff and pelletized.

Rats were divided into four groups: one was administered with physiological saline (sham) and the other three groups were injected with carcinogen DMH in combination with administration with basic feedstuff (PC), p13 (p13) and p14 (p14), respectively. The rats were fed ad libitum with purified water and basic foodstuff. The test materials were administered at a dose of 200 μg/kg for 8 weeks, during which body weight and feed intake were recorded once a week. Clinical symptoms were monitored with the naked eye (FIG. 14).

After the injection of DMH, as can be seen in FIG. 14, feed intake was significantly reduced in all the test groups compared to the control (sham). The reduction of feed intake by DMH was considered attributable to the DMH-induced development of polyps which led to decreased digestibility. Relatively high feed intake rateswere detected in the p14-administered group. Administration p14 alone allowed higher feed intake rates than administration with p13 alone, indicating that p14 is useful for the therapy of colorectal cancer.

4-2. Suppressive Activity of p14 against DMH-induced Polyps

To examine the suppressive effect of p14 on DMH-induced polyps, a postmortem examination was made on all subjects after administration of the test materials for 8 weeks. Polyps were examined on a predetermined size of the large intestine organ extending to the rectum above the anus. The excised large intestine tissue was maximally swelled in a mixture of 1:1 0.85% saline : 10% neutral formalin and then pre-fixed for 30 min in 10% neutral formalin. It was thinly spread and attached to a filter membrane so as not to distort the shape, followed by post-fixation in 10% neutral formalin. The fixed large intestine was stained with 0.5% methylene blue to visualize aberrant crypts (AC) and aberrant crypt foci (ACF) which were counted under a 100x optical microscope. The counts are shown in FIGS. 15a and 15b, respectively. As can be seen in FIGS. 15a and 15b, AC and ACF counts were significantly decreased in the p14 group compared to the other test groups.

EXAMPLE 5: Anticancer Activity of p14 in Xenograft Animal Model

The anticancer activity of p14 was assayed in xenografted animals which had been prepared by subcutaneously injecting 1x107 cells of the human colon cancer cell line DLD-1 into nude mice. To this end, first, 1x107 cells/nude mouse/100 μL was implanted into a subcutaneous region. Ten days after implantation, the state of the tumor cells was examined and the animals in which the implanted tumor cells had stable growth were monitored. Before the occurrence of central necrosis, the animals in which the tumors rapidly grew with a sufficient supply of blood were sacrificed. Peripheral tumor regions where cell division was taking place vigorously were excised to obtain tumor fragments in a predetermined size (3x3x3 mm). Subsequently, the tumor fragment was implanted by inserting a trocar carrying the tumor fragment into the mice through an incision made to a length of about 4 mm on the lateral flank of the left hind leg. In this context, the end of the trocar was allowed to reach the rear lateral flank of the left frontal leg. The trocar was withdrawn while being lightly and rapidly rotated, whereby the tumor fragment was set in the target position. The incision was aseptically treated. The position of the tumor fragment was confirmed by touching the surface of the skin. Two or more observations were made of the growth of the tumor fragment and animals in which the tumor fragment was successfully engrafted were selected for further experiments.

p14 was intravenously injected at a dose of 1 mg/kg four times in total (once per two days) into the xenografted animal models. The size of the tumor was measured every two days using a vernier caliper. From the measurements, the volume of tumor was calculated according to the following equation, and is shown in FIG. 16.

$$\text{Volume of Tumor} = (\text{Length of Long Axis x } (\text{Length of Short Axis})^2)/2$$

After four rounds of administration, the tumors were excised and photographed as shown in FIG. 17.

As can be seen in FIGS. 16 and 17, p14 was found to regulate, although not thoroughly, the growth of the tumor so that the size of tumor in the p14-administered group was remarkably reduced, compared to the control. The volume of the tumor was increased at a rate of 63.3% in the control and 49.6% (92.8→196.8mm2) in the p14-administered group, indicating that the percent inhibition of p14 was more than 10 %.

EXPERIMENTAL EXAMPLE 6: Anti-Allergic Activity of p14

A. Assay for Anti-Allergic Activity in Splenocytes

Cells were separated from the spleen of Balb/c mice, and the splenocytes were seeded at a density of 2x106 cells/mL/well into well plates. Treatment with 1 μg/mL Concanavalin A (ConA) allowed the splenocytes to be activated as in allergic diseases. The cells were incubated for 48 hours with various concentrations of the target proteins p13 and p14. After centrifugation, the supernatant thus formed was subjected to ELISA assay. For control comparison, the splenocytes were treated with PBS devoid of p14. In the case of the quantitative analysis of IL-4, the cells were incubated in the presence of a mixture of 1:1 p13 : p14 (MIX), as well. INF-γ and IL-4, which are respectively representative of the cytokines of T helper (Th1) and Th2 cells, were quantitatively analyzed. The results are shown in FIGS. 18a and 18b, respectively. As seen in FIGS. 18a and 18b, the level of IL-4 was slightly reduced in the p14-administred group compared to the group treated with Con A alone while the level of INF-γ was increased by p14. This data indicates that the protein p14 of the present invention exhibits anti-allergic activity by increasing the level of INF-γ and inhibiting the production of IL-4.

B. Assay of p14 for Anti-Allergic Activity Using Mouse Macrophage Cells

To confirm the anti-allergic activity of the lactic acid bacteria-derived p14 of the present invention, the production of NO by mouse macrophages (Raw 264.7) treated with the p14 was examined. The production of NO by macrophages is used as a parameter indicative of anti-allergic activity in terms of the activity of immune cells. For this reason, Raw 264.7 was utilized in producing NO. Raw 264.7 cells were seeded at a density of 5x105 cells/well into well plates and treated with LPS (lipopolysaccharide) concurrently with various concentrations of the recombinant protein p14 (10, 100, 1000 ng/mL). The production amount of NO was measured at different times post-treatment (24H: FIG. 19a, 48H: FIG. 19b, 72H: FIG 19c). As can be seen in FIGS. 19a to 19c, the production amount of NO was found to peak in the group treated with the His tag-removed p14. Singularly, pure p14 (p14-E) free of the His tag induced the production of NO at a higher level than did p14 conjugated with the His tag. This result demonstrates that pure p14 free of His tag induces anti-allergic activity in mouse macrophages (Raw 264.7).

C. Assay of p14 for Anti-Allergic Activity in NC/Nga Mice

For use in an in vivo assay for confirming the anti-allergic activity of the lactic acid bacteria-derived p14 of the present invention, the hair of NC/Nga mice, which are an animal model for atopic dermatitis, was cut off from the neck down to the tail using hairclippers and a deplilatory. For one day after shaving, the mice were left lest they might be affected by skin diseases and injuries which would be generated during the shaving procedure, other than atopic dermatitis. Each mouse was challenged eight times in total with 200 μL of a solution of 1% DNCB in a mixture of 1:3 acetone : olive oil to induce atopic dermatitis.

After the induction of atopic dermatitis, p13 or p14 was intraperitoneally injected at a dose of 400 μg into the NC/Nga mice twice a week for four weeks. A sham group was used as a negative control (NC) while for a positive control (PC), NC/Nga mice were treated with DNCB, but without the protein. During the experiment for four weeks, ocular blood was taken every week from the mice.

Four weeks later, the mice were sacrificed and the affected areas were shaved to examine the progress of atopic dermatitis with the naked eye. The atopic dermatitis had been improved in both the p13- and the p14-administered group, compared to the positive group (FIG. 20). In addition, the mouth and the area where the front legs could not reach (the front legs, lateral sides and tail) were in a better condition in the p14-administered group than the p13-administered group. p14 was found to be have a greater therapeutic effect on atopic dermatitis than p13, as observed with the naked eye. The secondary inflammation caused by scratching due to itching was significantly reduced by the administration of p14.

Many international scientific journals had it that the level of immunoglobulin E (IgE) is exacerbated in patients with atopic dermatitis, compared to healthy persons. In practical clinics, agents for reducing IgE levels are used. Hence, IgE is known as an index for allergy therapy. Cytokines secreted from Th2, such as IL-4, IL-5 and IL-13, are reported to stay at higher levels in atopic dermatitis patients than healthy persons. Together with IgE, IL-4, representative of the cytokines of Th2, is used as a main index for atopic dermatitis. Serum IgE levels were measured by enzyme-linked immunoserological assay (ELISA). Serum IgE levels were found to significantly decrease in both the p13- and the p14-administered group, compared to the positive control (FIG. 21), as measured by ELISA.

Serum IL-4 levels were compared in the same manner as described above. Both the p13- and p14-administered group had lower serum IL-4 levels, compared to the positive control (FIG. 22). Particularly, the serum IL-4 level in the p14-administered group was lower than that in the p13-administered group. Taken together, the data obtained above demonstrate that the protein p14 of the present invention has therapeutic activity for atopic dermatitis in vivo.

## Claims

**1.** A pharmaceutical composition for use in the treatment of allergy, comprising:

(a) the protein consisting of the amino acid sequence of SEQ ID NO: 1 or 3; and
(b) a pharmaceutically acceptable excipient, diluent, carrier or buffer.

**2.** The pharmaceutical composition for use in the treatment of allergy of claim 1, wherein the protein is derived from *Lactobacillus casei, Lactobacillus paracasei* or *Lactobacillus rhamnosus.*

3. The pharmaceutical composition for use in the treatment of allergy of claim 1, wherein the protein has a molecular weight of 14 kDa.

## Patentansprüche

1. Pharmazeutische Zusammensetzung zur Verwendung bei der Behandlung von Allergie, umfassend:

   (a) das Protein, bestehend aus der Aminosäuresequenz von SEQ ID Nr. 1 oder 3; und
   (b) einen pharmazeutisch annehmbaren Hilfsstoff, Verdünner, Träger oder Puffer.

2. Pharmazeutische Zusammensetzung zur Verwendung bei der Behandlung von Allergie nach Anspruch 1, wobei das Protein von *Lactobacillus casei, Lactobacillus paracasei* oder *Lactobacillus rhamnosus* stammt.

3. Pharmazeutische Zusammensetzung zur Verwendung bei der Behandlung von Allergie nach Anspruch 1, wobei das Protein ein Molekulargewicht von 14 kDa aufweist.

## Revendications

1. Composition pharmaceutique destinée à être utilisée dans le traitement de l'allergie, comprenant :

   (a) la protéine consistant en la séquence d'acides aminés de la séquence SEQ ID NO : 1 ou 3 ; et
   (b) un excipient, un diluant, un support ou un tampon pharmaceutiquement acceptable.

2. Composition pharmaceutique destinée à être utilisée dans le traitement de l'allergie de la revendication 1, dans laquelle la protéine est dérivée de *Lactobacillus casei,* de *Lactobacillus paracasei* ou de *Lactobacillus rhamnosus.*

3. Composition pharmaceutique destinée à être utilisée dans le traitement de l'allergie de la revendication 1, dans laquelle la protéine a un poids moléculaire de 14 kDa.

[Fig. 1]

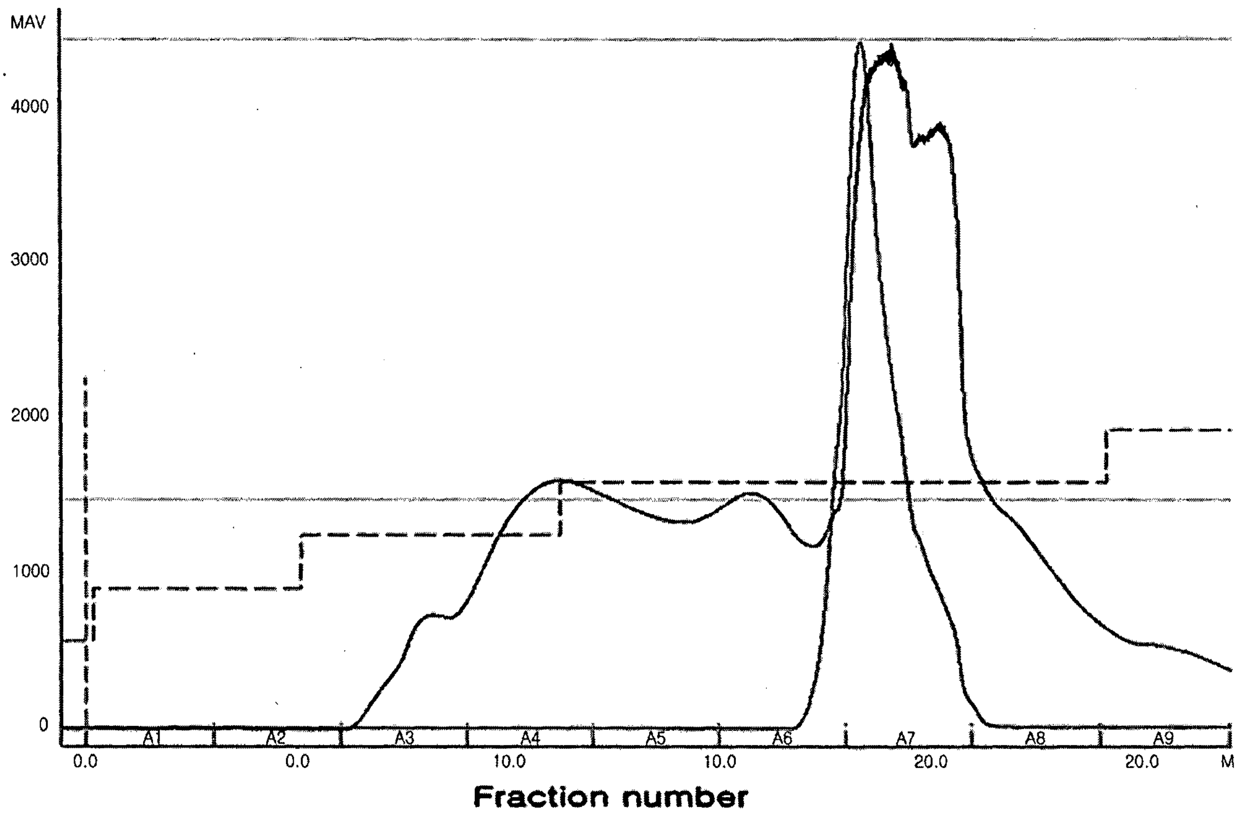
MAV
4000
3000
2000
1000
0
A1
A2
A3
A4
A5
A6
A7
A8
A9
0.0
0.0
10.0
10.0
20.0
20.0
M
Fraction number

[Fig. 2]

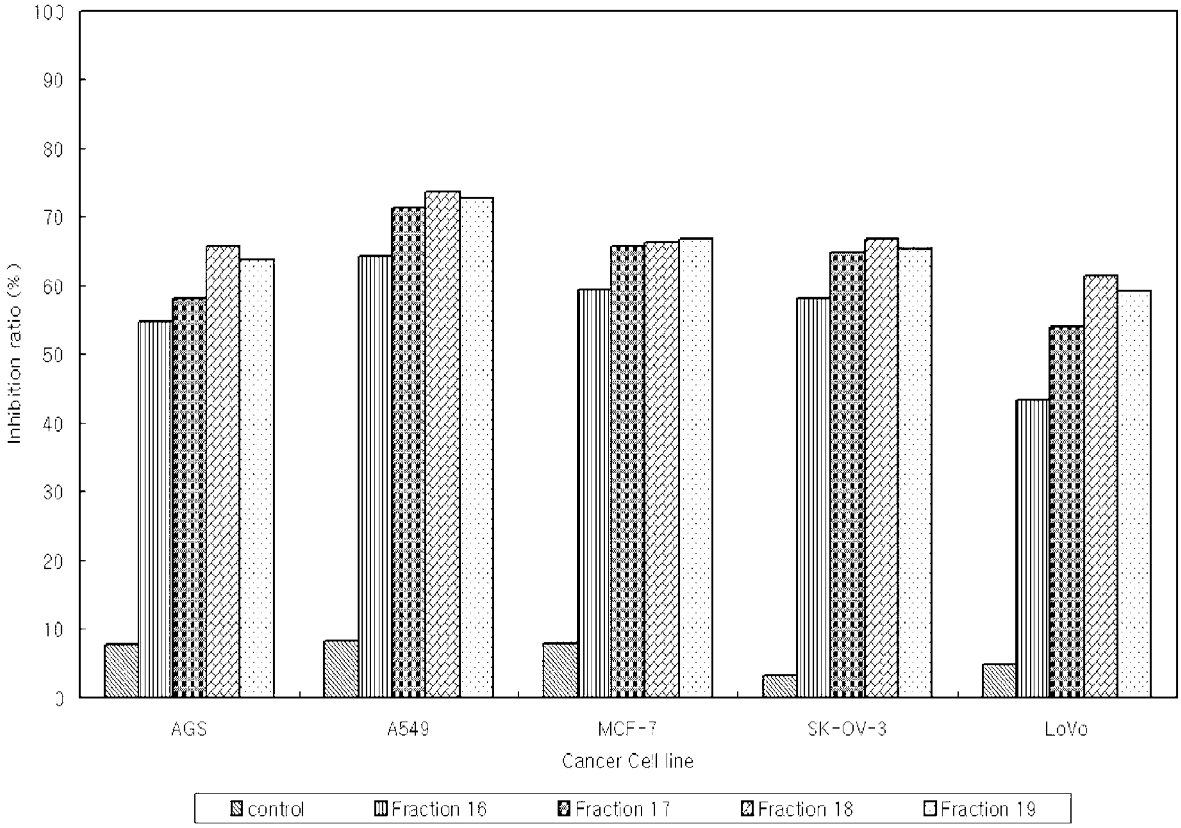
100
90
80
70
60
50
40
30
20
10
0
Inhibition ratio (%)
AGS
A549
MCF-7
SK-OV-3
LoVo
Cancer Cell line
control
Fraction 16
Fraction 17
Fraction 18
Fraction 19

[Fig. 3]

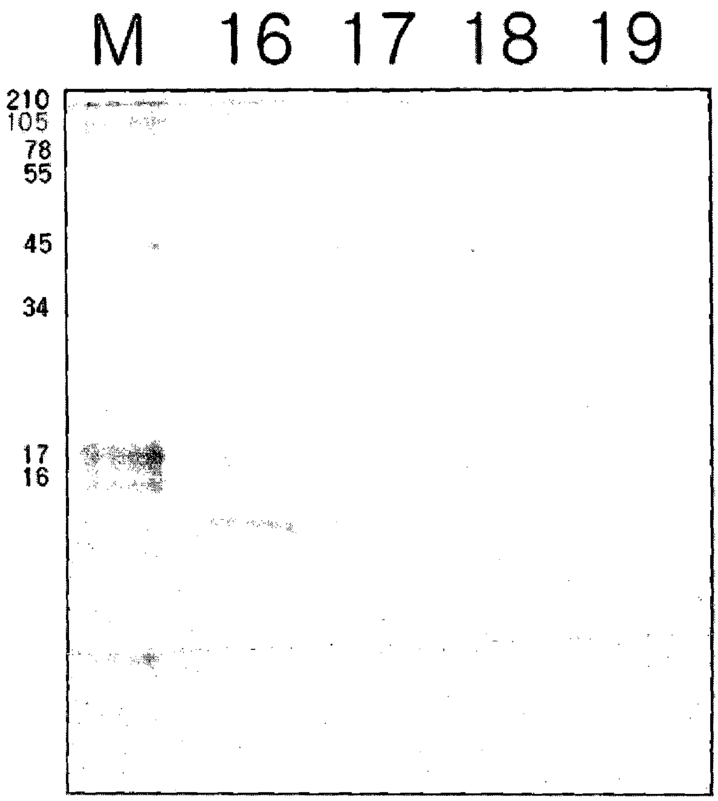


[Fig. 4]

| Reference protein | Peptide sequence | z | Score XC | Molecular weight (Da) |
|---|---|---|---|---|
| Cell wall-associated hydrolase [Lactobacillus casei ATCC 334) | | | 30.26 | 49,407 |
| | K.IQAVISIAEQQIGK.P | 2 | 3.48 | 1,105 |
| | K.IQAVISIAEQQIGK.P | 2 | 4.04 | 1,371 |
| | K.YTALAYFMPDFAVR.P | 2 | 4.60 | 1,316 |
| | K.YTALAYFMPDFAVRPSL. | 2 | 5.17 | 1,163 |
| Hypothetical protein LSEI_1559 | | | 10.23 | 14,283 |
| | K.ILADELGLTVPK.I | 2 | 4.68 | 1,299 |
| Hypothetical protein LSEI_0419 | | | 10.22 | 12,856 |
| | K.AIGDNPASELLVFYDLGS.AK.M | 2 | 4.31 | 883 |

[Fig. 5a]

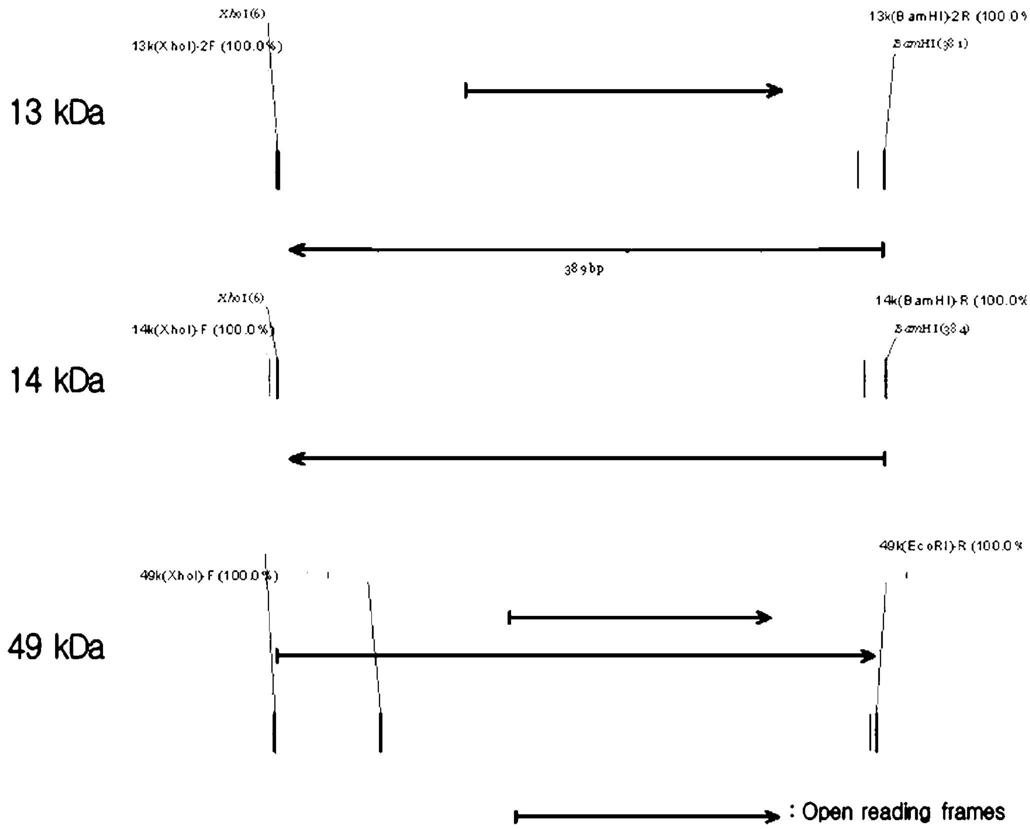
XhoI(6)
13k(XhoI)-2F (100.0%)
13k(BamHI)-2R (100.0%
13 kDa
389bp
XhoI(6)
14k(XhoI)-F (100.0%)
14k(BamHI)-R (100.0%
14 kDa
49k(XhoI)-F (100.0%)
49k(EcoRI)-R (100.0%
49 kDa
: Open reading frames

[Fig. 5b]

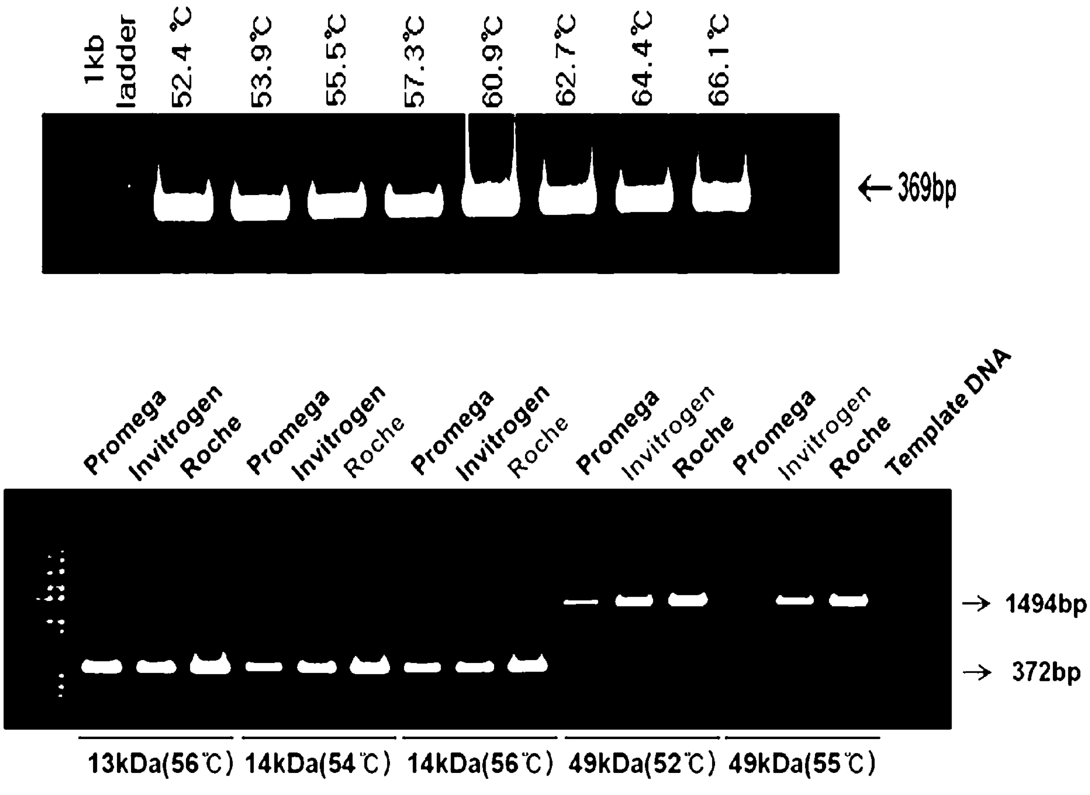
1kb ladder
52.4 ℃
53.9℃
55.5℃
57.3℃
60.9℃
62.7℃
64.4℃
66.1℃
← 369bp
Promega
Invitrogen
Roche
Promega
Invitrogen
Roche
Promega
Invitrogen
Roche
Promega
Invitrogen
Roche
Promega
Invitrogen
Roche
Template DNA
→ 1494bp
→ 372bp
13kDa(56℃)
14kDa(54℃)
14kDa(56℃)
49kDa(52℃)
49kDa(55℃)

[Fig. 6]

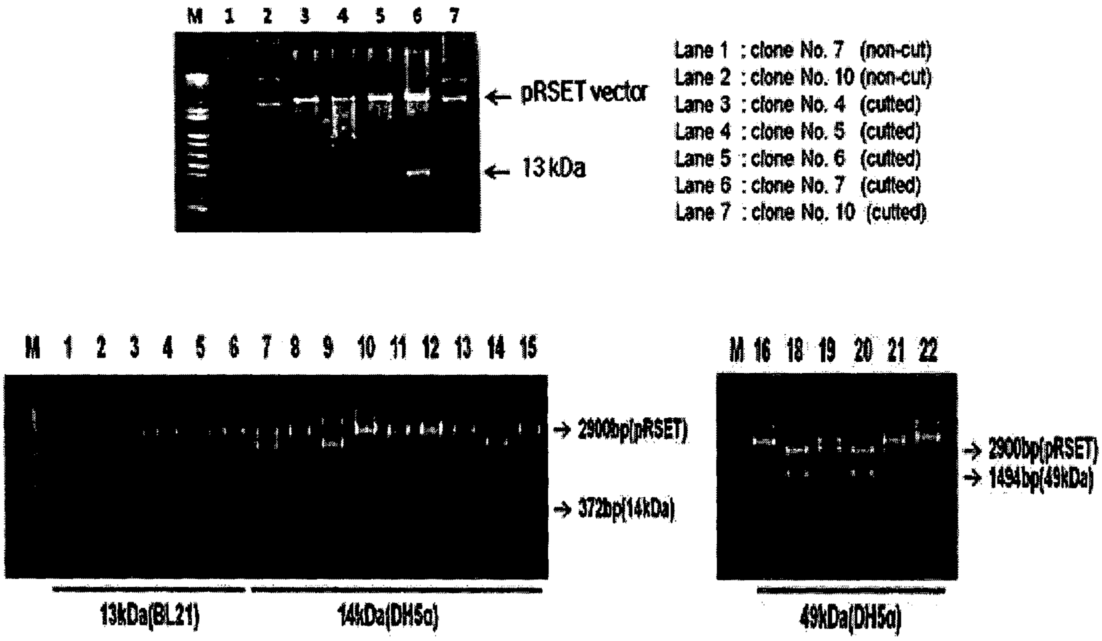
M 1 2 3 4 5 6 7
← pRSET vector
← 13 kDa
Lane 1 : clone No. 7 (non-cut)
Lane 2 : clone No. 10 (non-cut)
Lane 3 : clone No. 4 (cutted)
Lane 4 : clone No. 5 (cutted)
Lane 5 : clone No. 6 (cutted)
Lane 6 : clone No. 7 (cutted)
Lane 7 : clone No. 10 (cutted)
M 1 2 3 4 5 6 7 8 9 10 11 12 13 14 15
→ 2900bp(pRSET)
→ 372bp(14kDa)
13kDa(BL21)
14kDa(DH5α)
M 16 18 19 20 21 22
→ 2900bp(pRSET)
→ 1494bp(49kDa)
49kDa(DH5α)

[Fig. 7]

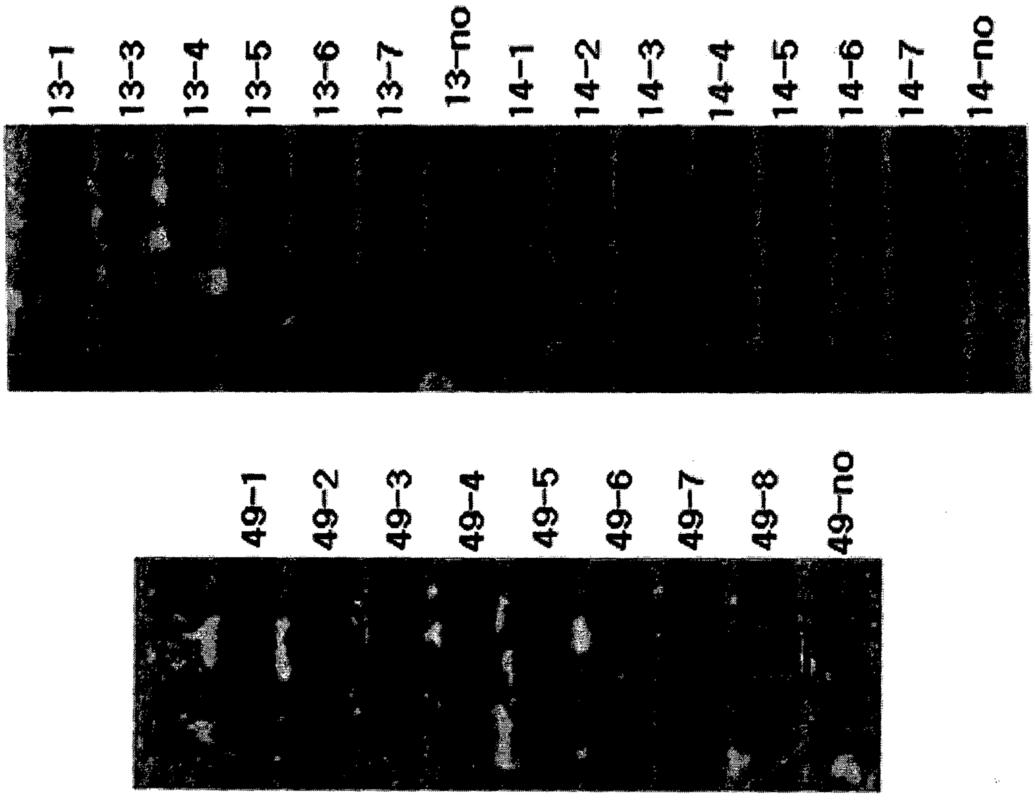
13-1
13-3
13-4
13-5
13-6
13-7
13-no
14-1
14-2
14-3
14-4
14-5
14-6
14-7
14-no
49-1
49-2
49-3
49-4
49-5
49-6
49-7
49-8
49-no

[Fig. 8]

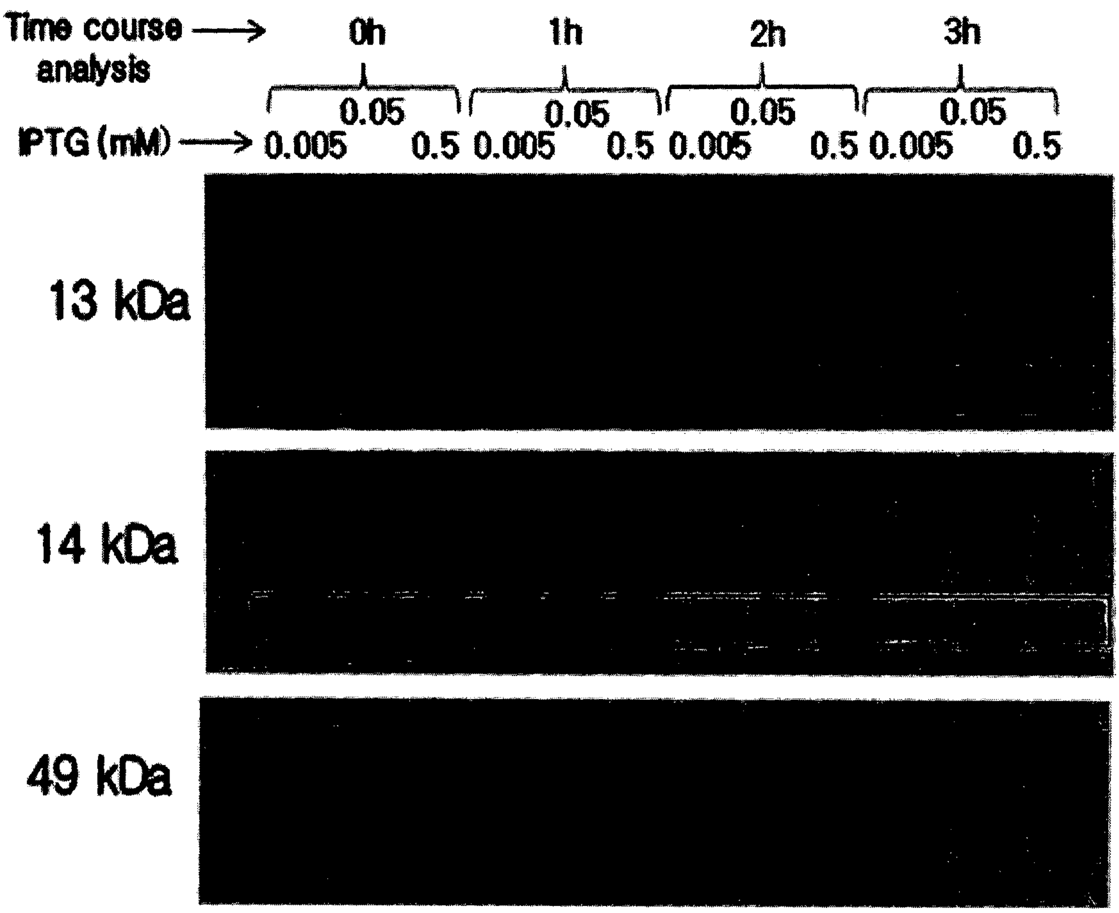
Time course analysis
0h
1h
2h
3h
IPTG (mM)
0.005
0.05
0.5
0.005
0.05
0.5
0.005
0.05
0.5
0.005
0.05
0.5
13 kDa
14 kDa
49 kDa

[Fig. 9a]

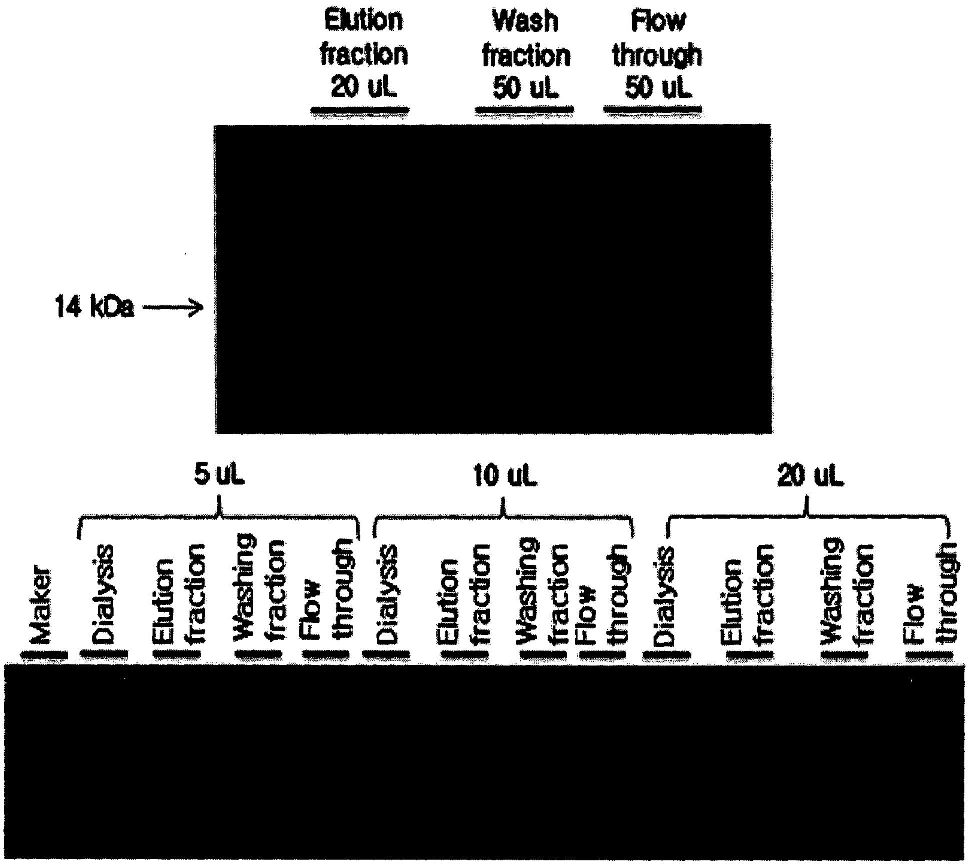
Elution fraction 20 uL
Wash fraction 50 uL
Flow through 50 uL
14 kDa
5 uL
10 uL
20 uL
Maker
Dialysis
Elution fraction
Washing fraction
Flow through
Dialysis
Elution fraction
Washing fraction
Flow through
Dialysis
Elution fraction
Washing fraction
Flow through

[Fig. 9b]

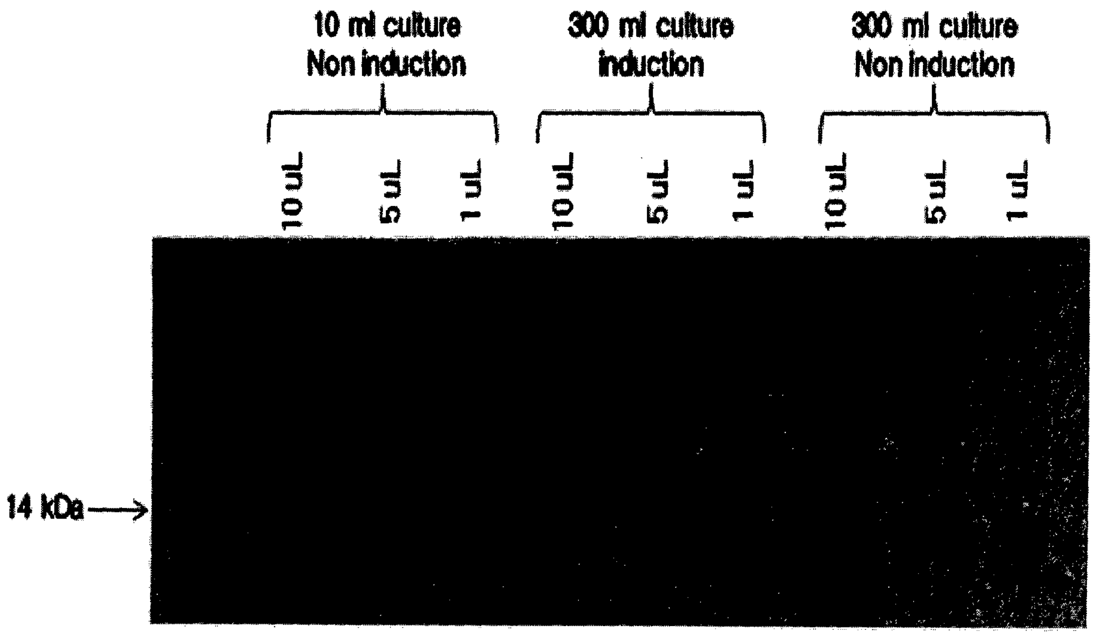
10 ml culture Non induction
300 ml culture induction
300 ml culture Non induction
10 uL
5 uL
1 uL
10 uL
5 uL
1 uL
10 uL
5 uL
1 uL
14 kDa

[Fig. 10]

**p13**

```
               (1)   30        40        50        60        70
p13            (1) AKDVPITTAGGTSDGKIGTDLDHIQKAIGDNPASELLVFYDLGSAKMNLDI
p13QTOP-1      (1) --DVPITTAGGTSDGKIGTDLDHIQK------------------------
p13QTOP-2      (1) -------------------------KAIGDNPASELLVFYDLGSAKM----
Consensus      (1)   DVPITTAGGTSDGKIGTDLDHIQKAIGDNPASELLVFYDLGSAKM
```

**p14**

```
               (1)    80        90        100       110          123
p14            (1) 'DYENGRIANIMWDMDKIKTREEAMKILADELGLTVPKIVMRKIDEQVF
p14QTOP-1      (1) ------IANIMWDMDK--------------------------------
p14QTOP-2      (1) ------------------------KILADELGLTVPKI----------
p14QTOP-3      (1) -----RIANIMWDMDKIKT-----------------------------
Consensus      (1)      RIANIMWDMDKIKT     KILADELGLTVPKI
```

[Fig. 11a]

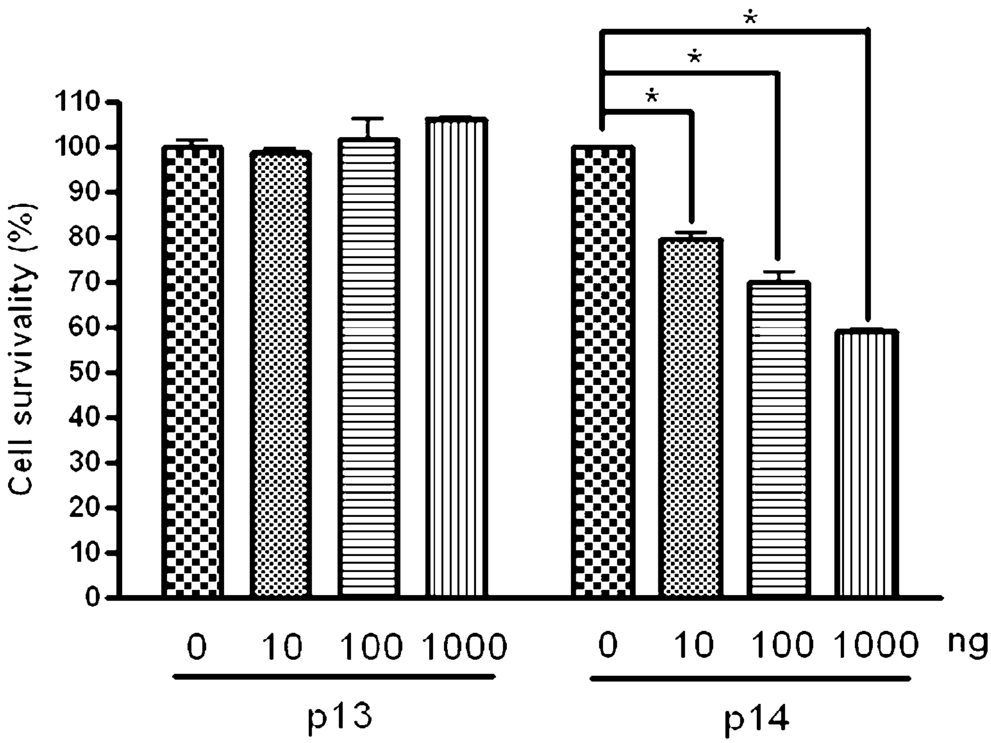

[Fig. 11b]

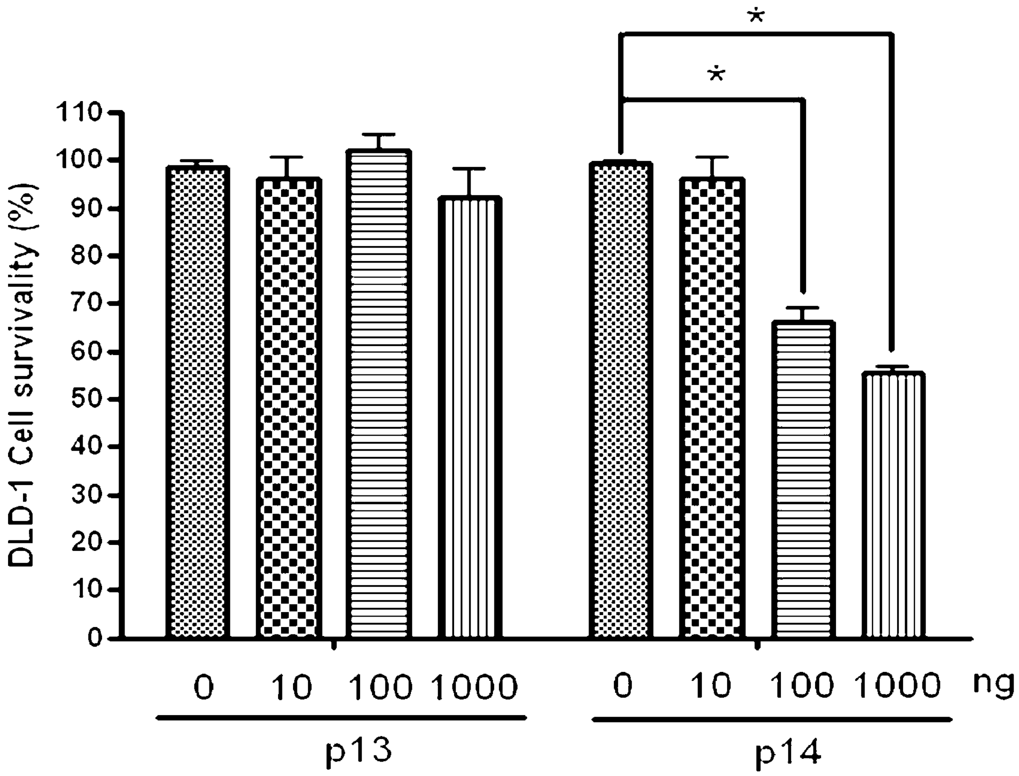


[Fig. 12]

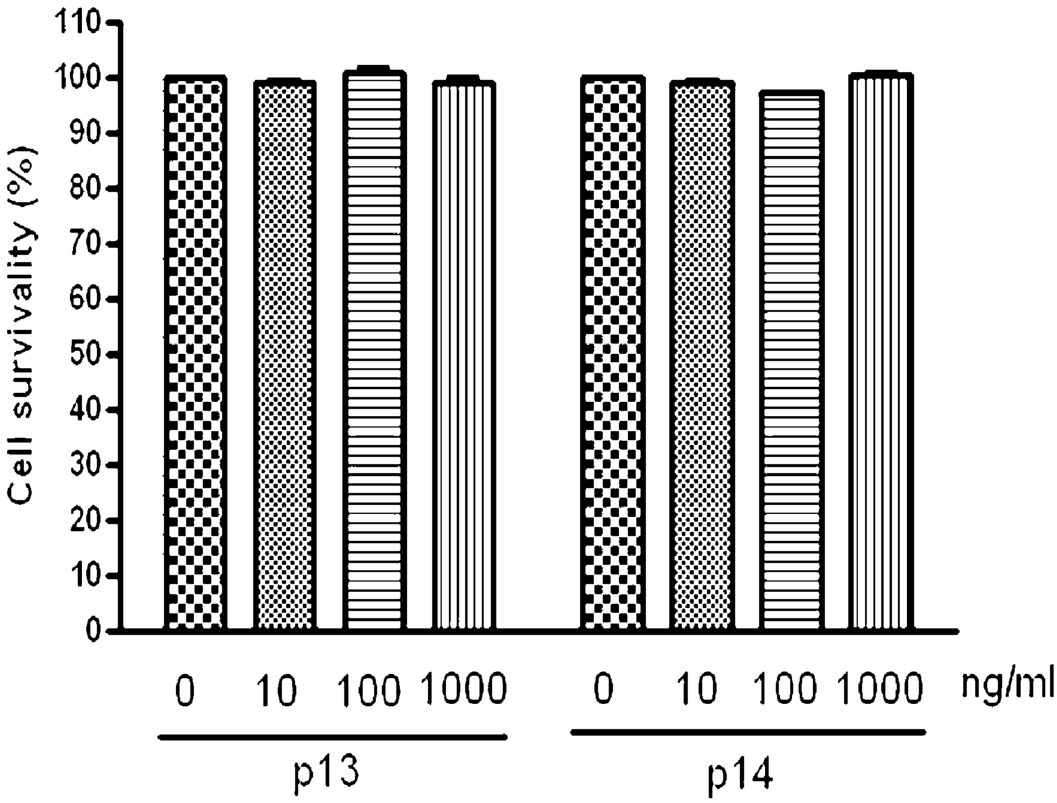

[Fig. 13a]

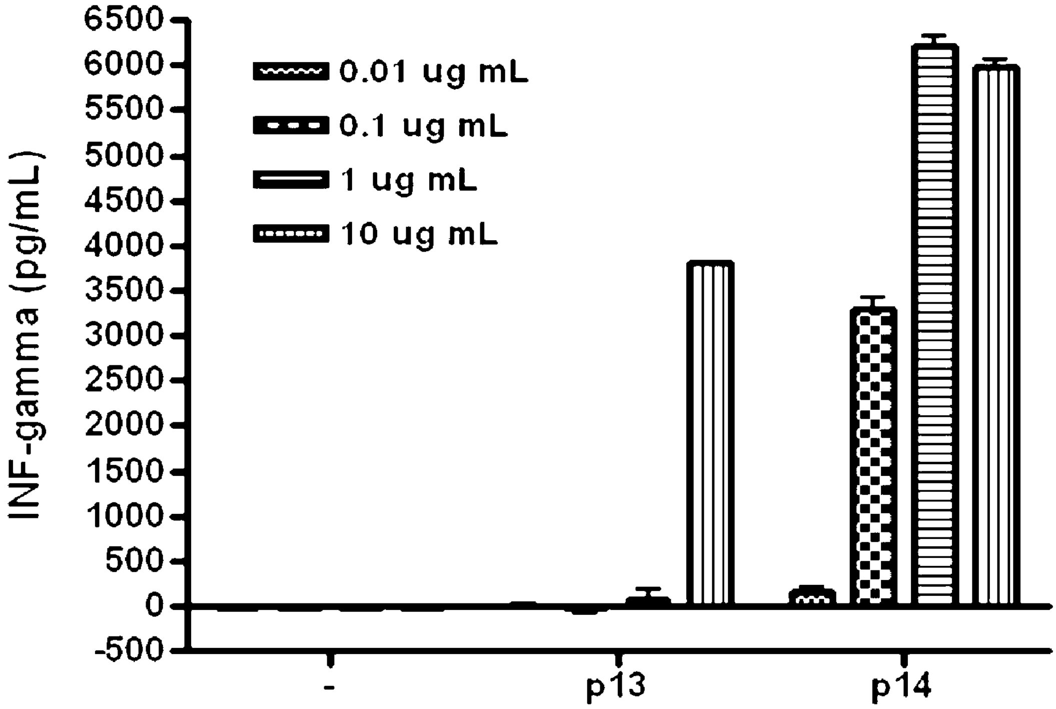


[Fig. 13b]

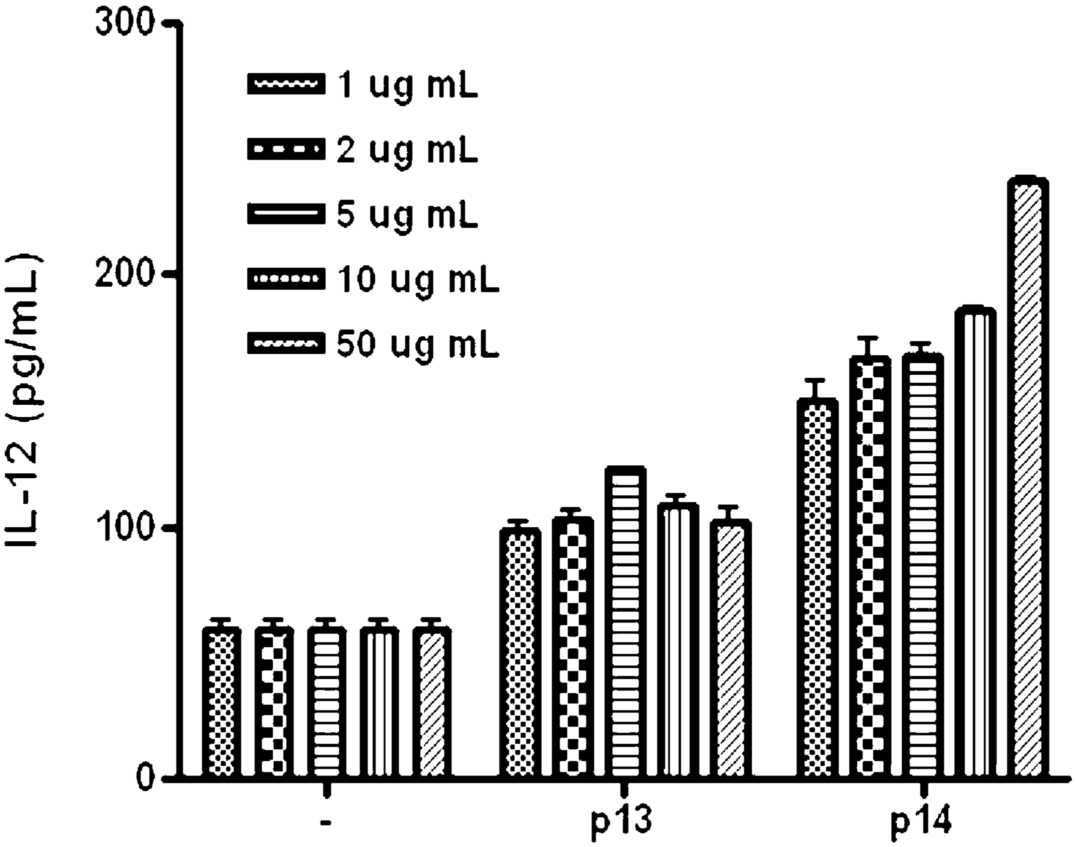

[Fig. 14]

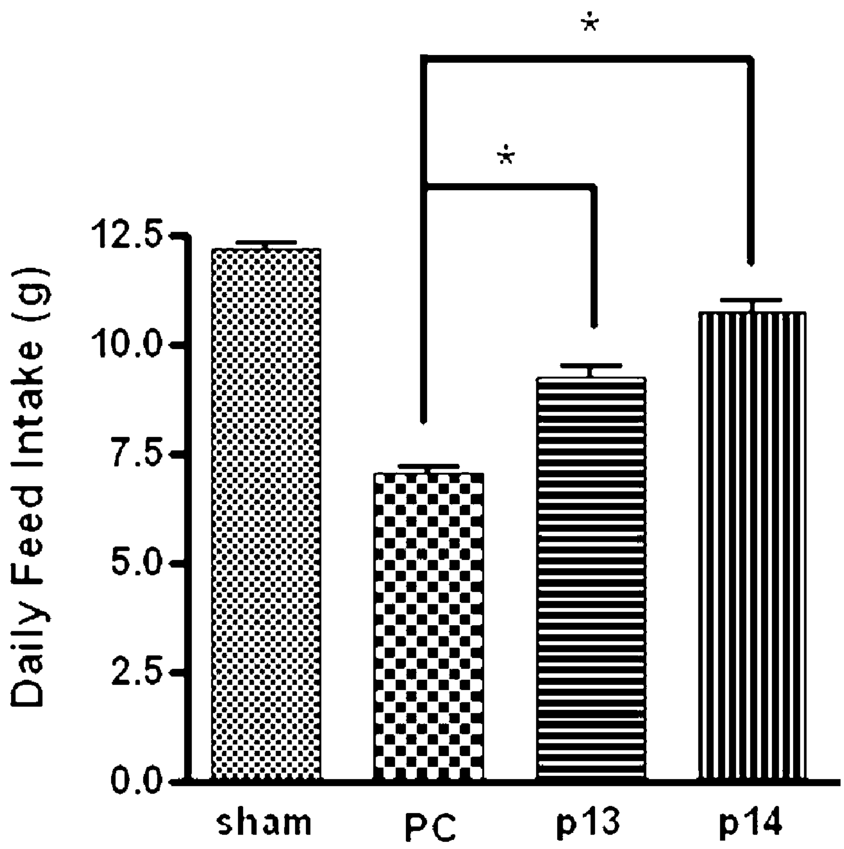


[Fig. 15a]

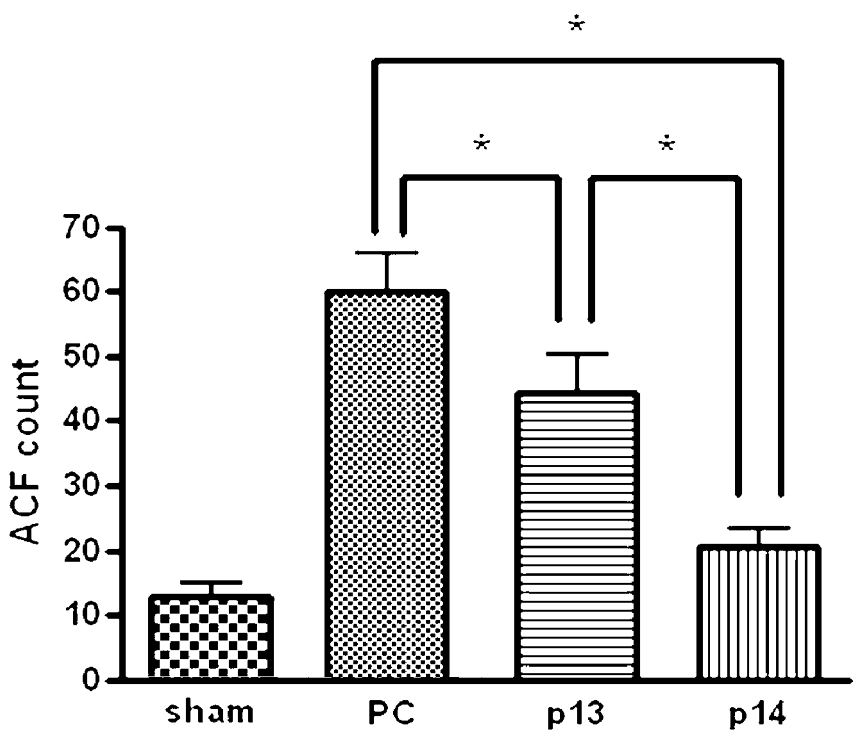

[Fig. 15b]

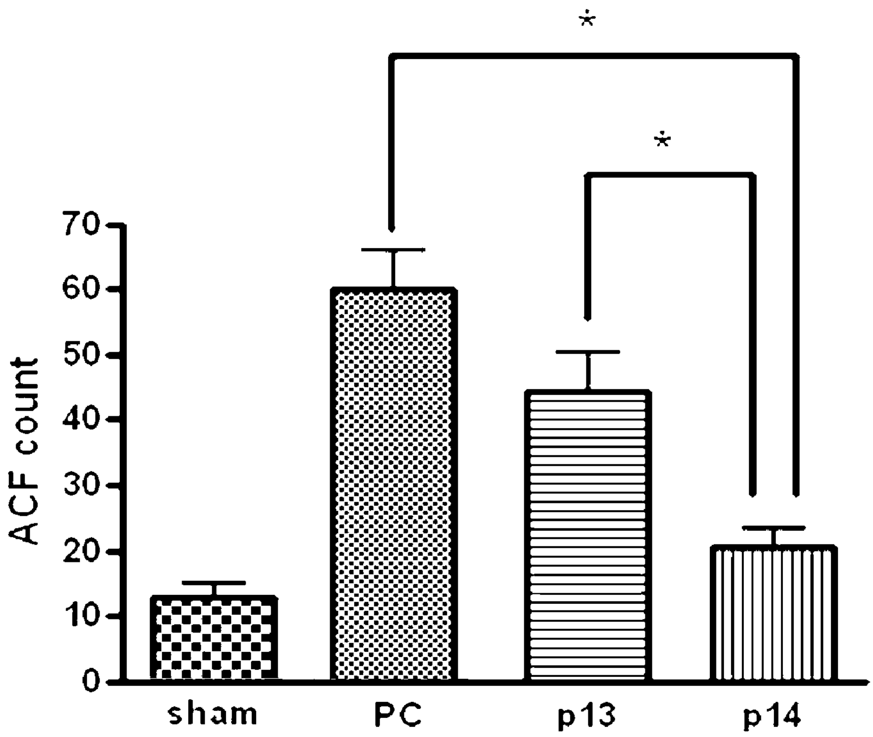


[Fig. 16]

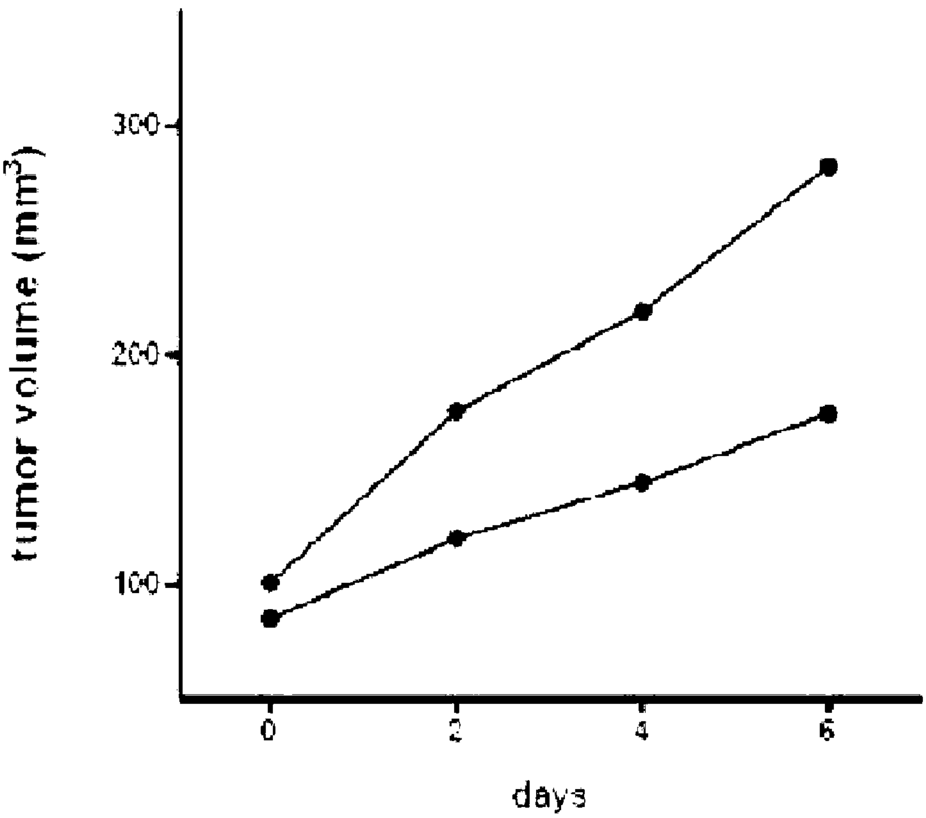

[Fig. 17]

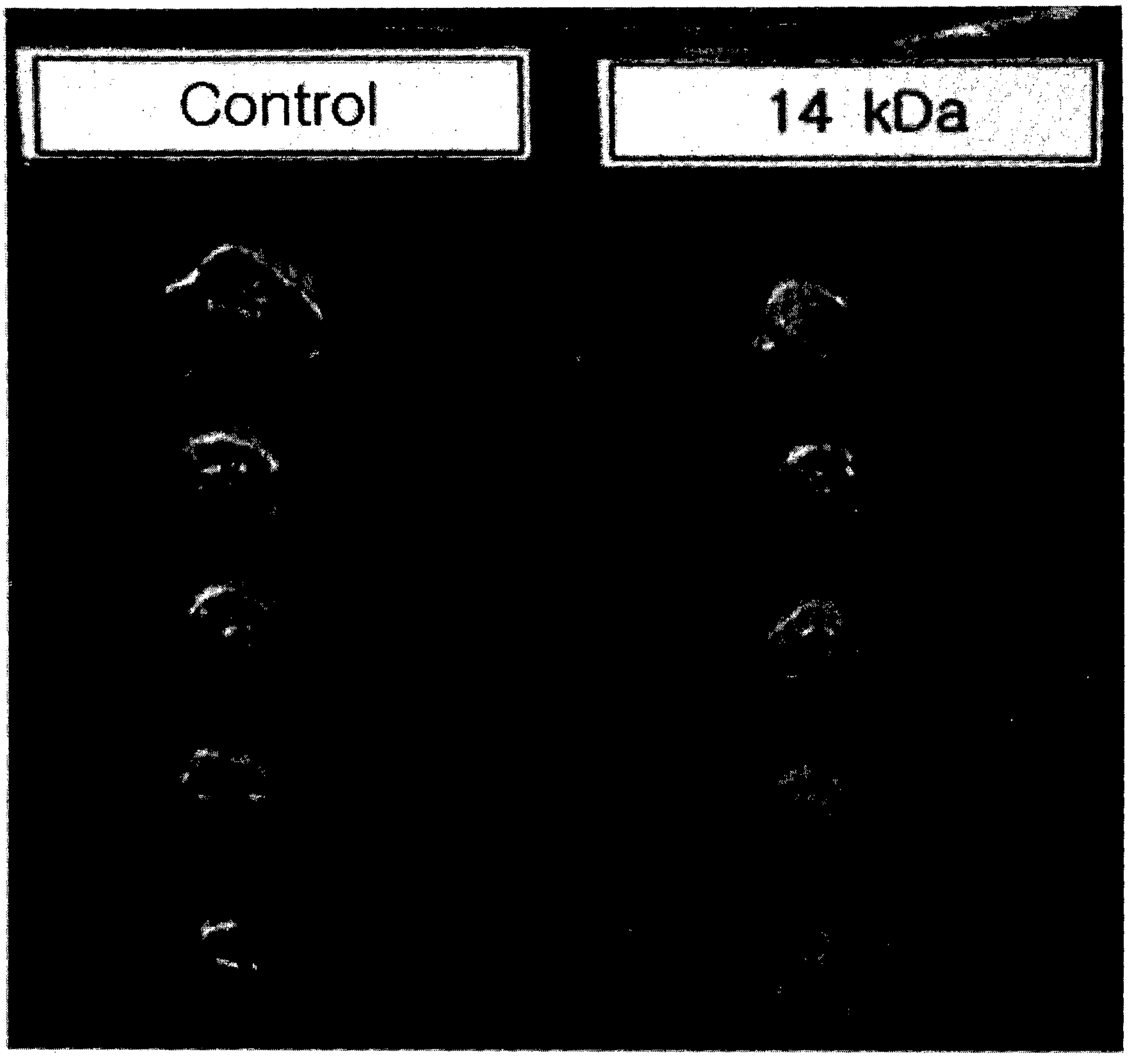

[Fig. 18a]

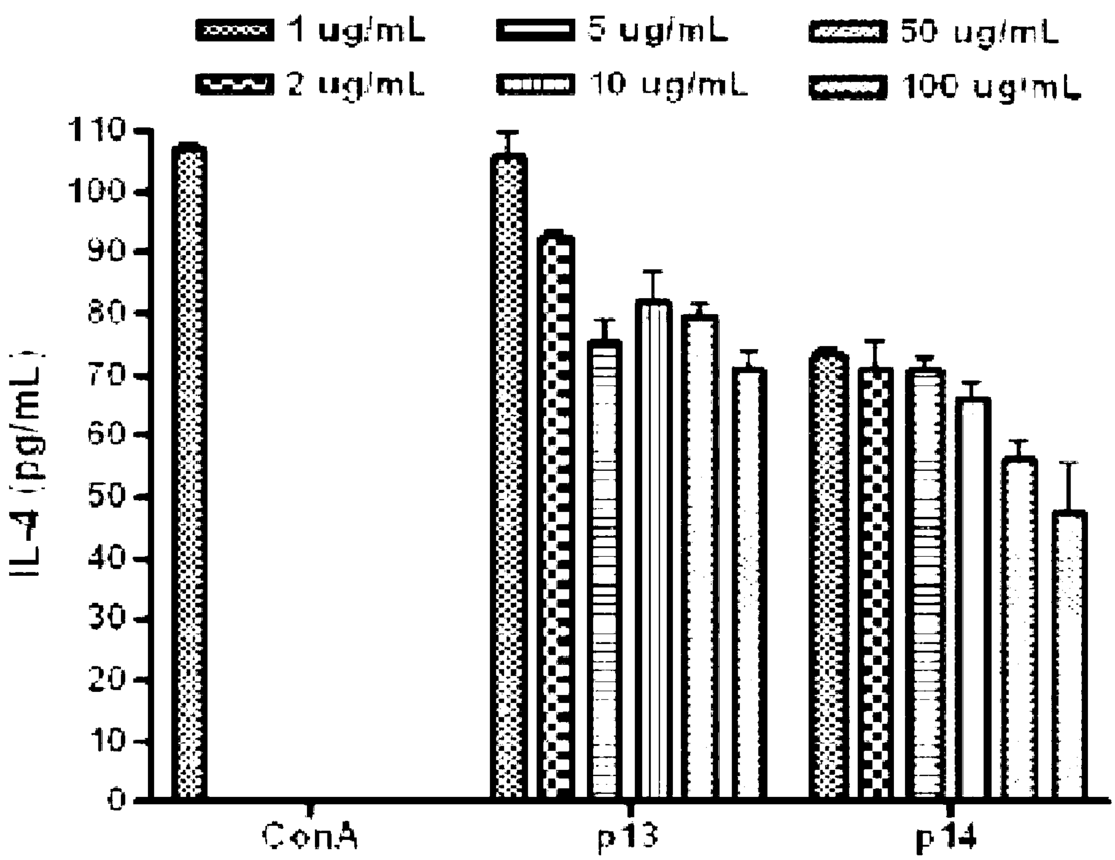


[Fig. 18b]

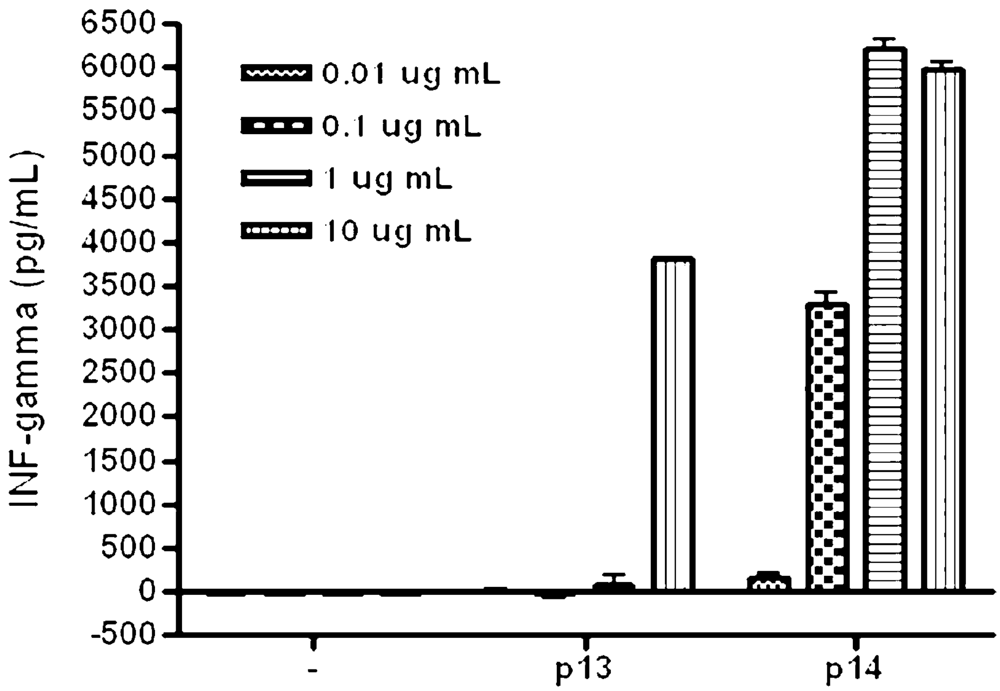

[Fig. 19a]

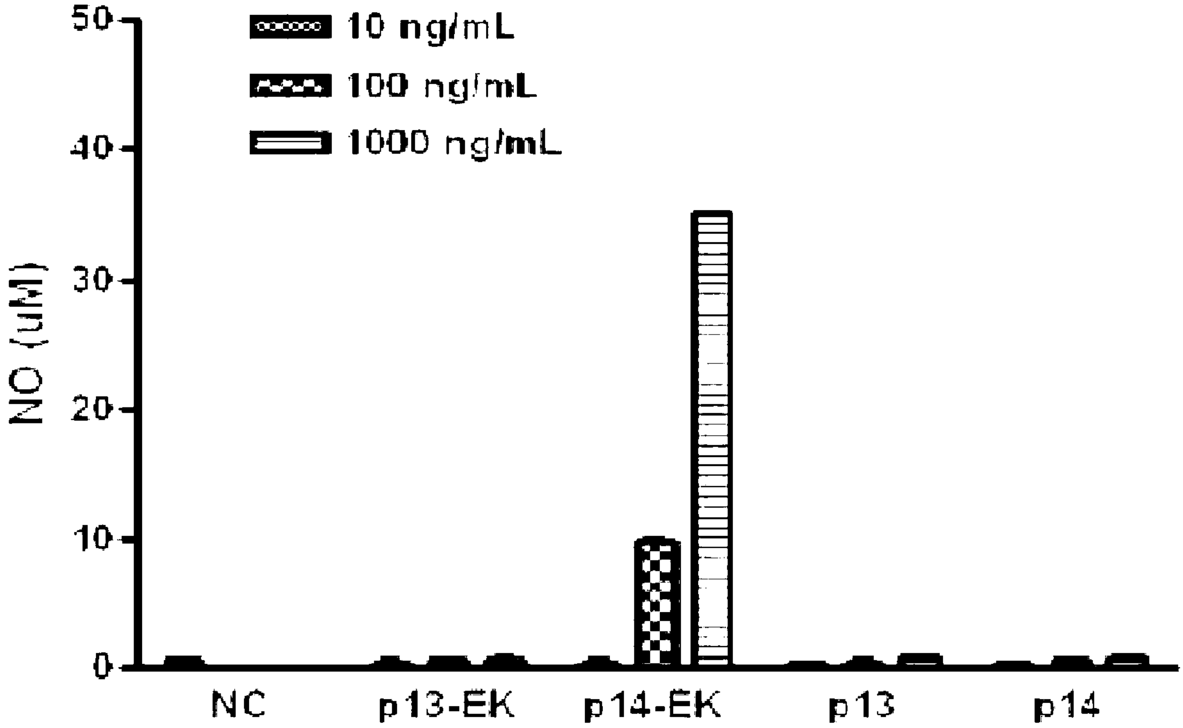


[Fig. 19b]

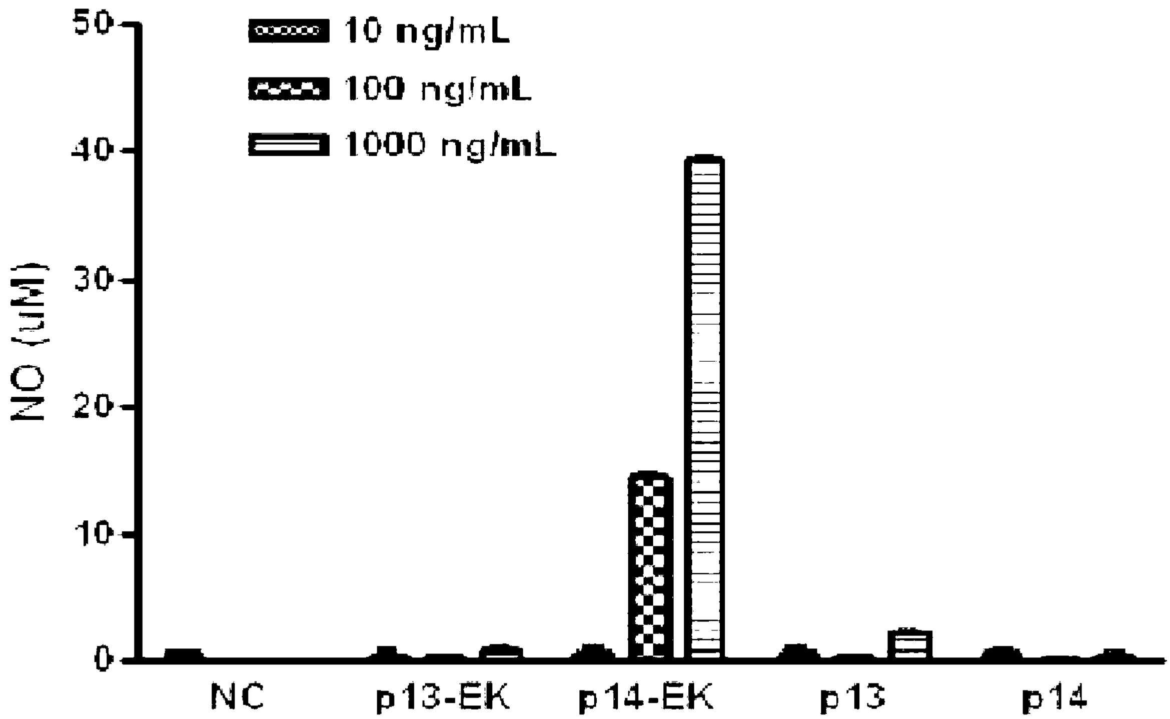

[Fig. 19c]

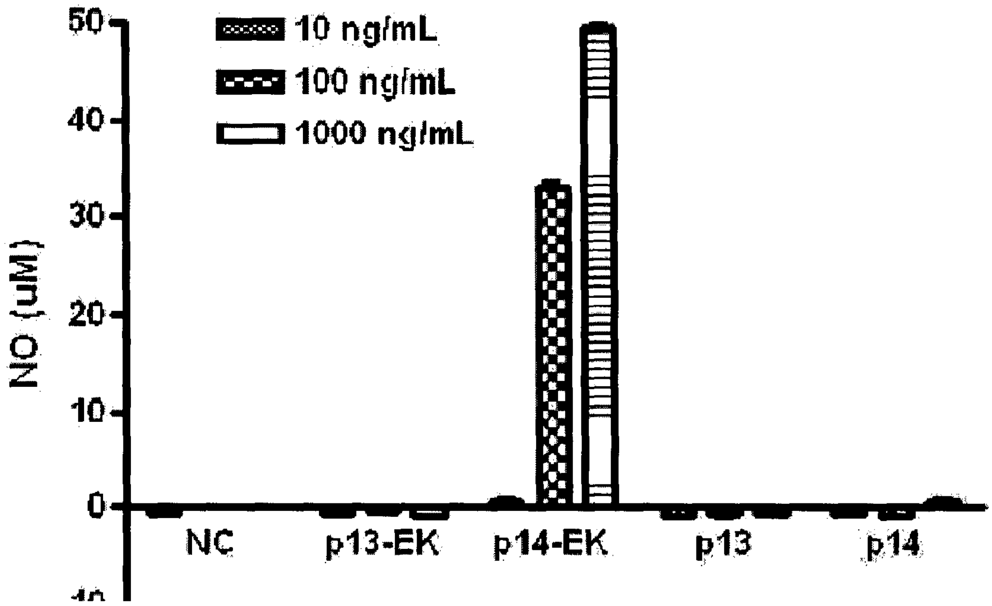


[Fig. 20]

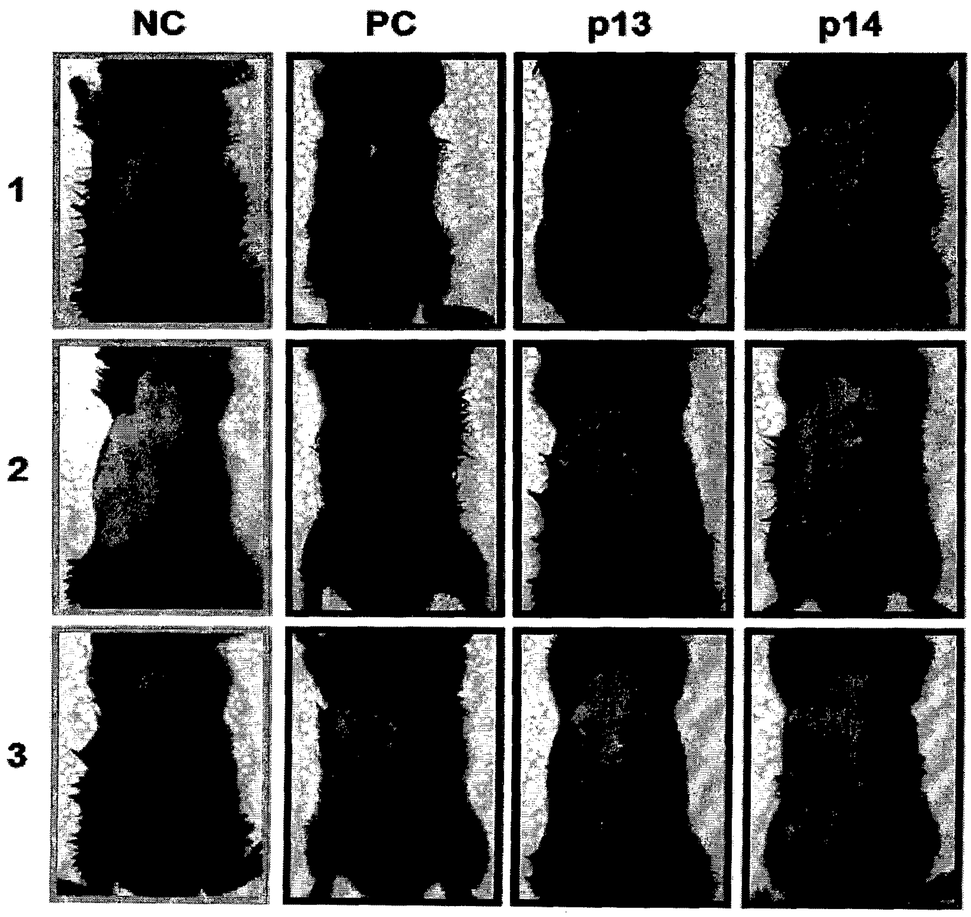

[Fig. 21]

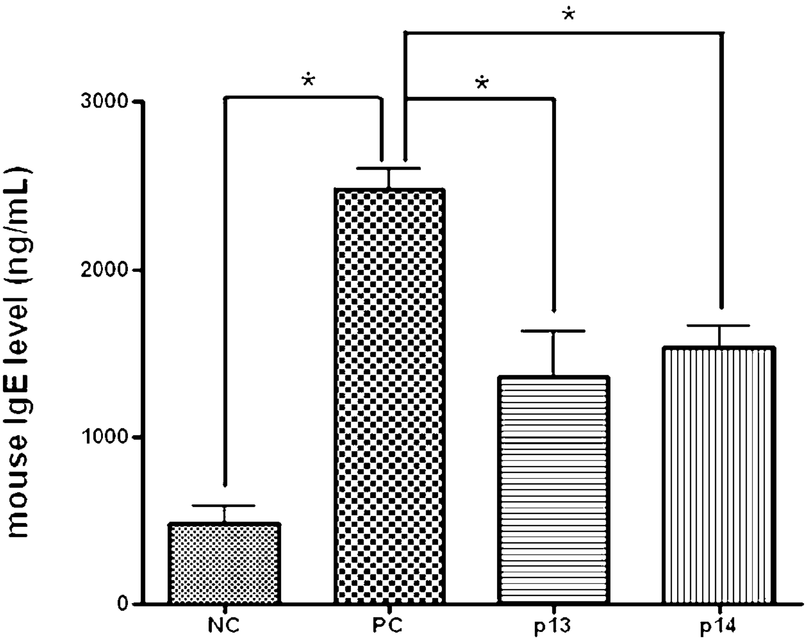
*
*
*
3000
2000
1000
0
mouse IgE level (ng/mL)
NC
PC
p13
p14

[Fig. 22]

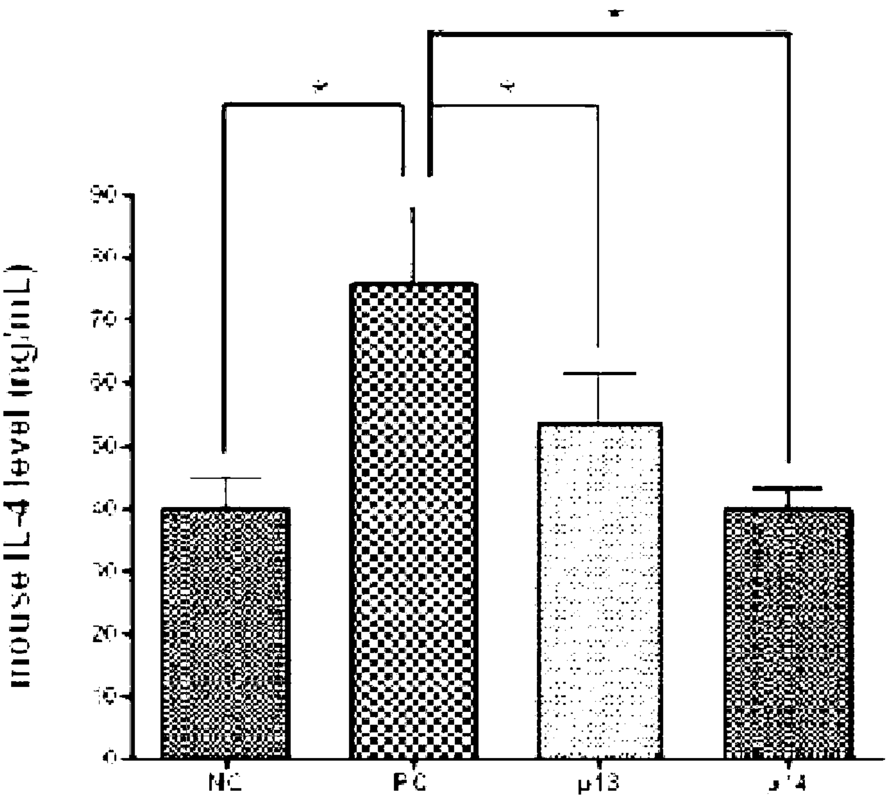
mouse IL-4 level (ng/mL)
NC
PC
p13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 307247 A
- WO 199108291 A

### Non-patent literature cited in the description

- **NABEL, E. G. et al.** *Science,* 1990, vol. 249, 1285-1288
- **WU et al.** *J. Bio. Chem.,* 1992, vol. 267, 963-967
- **WU ; WU.** *J. Bio. Chem.,* 1988, vol. 263, 14621-14624
- Remington's Pharmaceutical Sciences. 1995

<110> CELL BIOTECH CO., LTD.

<120> Anti-allergic Protein P14 and Anti-allergic Pharmaceutical Composition comprising the same

<130> P11-CBT-05

<160> 4

<170> KopatentIn 2.0

<210> 1
<211> 123
<212> PRT
<213> Lactobacillus casei

<400> 1

```
Met Ala Lys Ser Gln Asp Gln Phe Asn Glu Lys Val Gly Lys Glu Ile
 1               5                  10                  15

Asn Val Ser Asp Glu Ala Val Asp Lys Ala Ala Ala Gln Ile Glu Lys
            20                  25                  30

Val Gly Tyr Val Thr Glu Lys Asp Val Pro Glu Met Ile Asp Arg Asp
        35                  40                  45

Tyr Thr Arg Ala Leu Ser Lys Lys Val Ser Ala Lys Leu His Lys Asp
    50                  55                  60

Asn Asp Asp Asp Tyr Phe Tyr Glu Glu Pro Phe Asp Tyr Glu Asn Gly
65                  70                  75                  80

Arg Ile Ala Asn Ile Met Trp Asp Met Asp Lys Ile Lys Thr Arg Glu
                85                  90                  95

Glu Ala Met Lys Ile Leu Ala Asp Glu Leu Gly Leu Thr Val Pro Lys
            100                 105                 110

Ile Val Met Arg Lys Ile Asp Glu Gln Val Phe
        115                 120
```

<210> 2
<211> 372
<212> DNA
<213> Lactobacillus casei

<400> 2

```
atggcaaaat ctcaagacca attcaatgaa aaggtcggta aagaaattaa cgtttctgac      60

gaggccgtcg ataaagcagc tgcccaaatc gagaaagtcg gttacgtcac tgaaaaggac     120

gttccggaaa tgatcgatcg cgattacact cgggcattgt ccaagaaggt ttcggcaaaa     180

cttcacaaag acaatgacga tgattacttc tacgaagagc cttttgacta tgaaaatggc     240

cgcattgcaa atatcatgtg ggacatggat aaaattaaga cccgcgaaga agccatgaaa     300

attttggctg atgaacttgg ccttaccgtt cctaagattg taatgcgtaa aatcgacgaa     360

caggtcttct ag                                                         372
```

<210> 3
<211> 123
<212> PRT

<213> Lactobacillus rhamnosus GG

<400> 3

```
Met Ala Lys Ser Gln Asp Gln Phe Asn Glu Lys Ala Gly Lys Lys Ile
1               5                   10                  15

Thr Val Ser Asp Glu Ala Val Asp Lys Ala Ala Lys Lys Ile Glu Gln
            20                  25                  30

Val Gly Tyr Val Thr Glu Lys Asp Val Pro Glu Met Ile Asp Arg Asp
        35                  40                  45

Tyr Thr Arg Ala Leu Ser Lys Lys Val Ser Ala Lys Leu His Gln Asp
    50                  55                  60

Lys Asp Asp Asp Tyr Phe Tyr Glu Glu Pro Phe Asp Tyr Glu Asn Gly
65                  70                  75                  80

Arg Ile Ala Asn Ile Ile Trp Asp Met Asp Lys Ile Lys Thr Arg Glu
                85                  90                  95

Glu Ala Met Lys Thr Leu Ala Asn Glu Leu Gly Leu Thr Val Pro Lys
            100                 105                 110

Ile Val Met Arg Lys Val Asp Glu Gln Val Phe
        115                 120
```

<210> 4
<211> 372
<212> DNA
<213> Lactobacillus rhamnosus

<400> 4

```
atggcaaaat ctcaagacca attcaatgaa aaggtcggta aagaaattaa cgtttctgac      60

gaggccgtcg ataaagcagc tgcccaaatc gagaaagtcg gttacgtcac tgaaaaggac     120

gttccggaaa tgatcgatcg cgattacact cgggcattgt ccaagaaggt ttcggcaaaa     180

cttcacaaag acaatgacga tgattacttc tacgaagagc cttttgacta tgaaaatggc     240

cgcattgcaa atatcatgtg ggacatggat aaaattaaga cccgcgaaga agccatgaaa     300

attttggctg atgaacttgg ccttaccgtt cctaagattg taatgcgtaa aatcgacgaa     360

caggtcttct ag                                                         372
```